(19) Europäisches Patentamt
European Patent Office
Office européen des brevets

(11) EP 3 815 739 A1

(12) **EUROPEAN PATENT APPLICATION**
published in accordance with Art. 153(4) EPC

(43) Date of publication:
**05.05.2021 Bulletin 2021/18**

(21) Application number: **19807330.6**

(22) Date of filing: **20.05.2019**

(51) Int Cl.:
*A61N 1/36* (2006.01)    *A61N 1/04* (2006.01)

(86) International application number:
**PCT/JP2019/019877**

(87) International publication number:
**WO 2019/225537 (28.11.2019 Gazette 2019/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.05.2018 JP 2018096745**
**26.06.2018 JP 2018120408**

(71) Applicant: **Fukae Technologies LLC**
**Takatsu-ku**
**Kawasaki-shi, Kanagawa 213-0033 (JP)**

(72) Inventor: **FUKAE, Kimitoshi**
**Kawasaki-shi Kanagawa 213-0033 (JP)**

(74) Representative: **Maiwald Patent- und Rechtsanwaltsgesellschaft mbH**
**Elisenhof**
**Elisenstraße 3**
**80335 München (DE)**

(54) **WATERPROOF CASE FOR LOW-FREQUENCY ELECTRIC STIMULATION DEVICE, WATERPROOF-TYPE LOW-FREQUENCY ELECTRIC STIMULATION DEVICE, AND INSULATOR ELECTRODE FOR LOW-FREQUENCY ELECTRIC STIMULATION**

(57)    [Problems to be Solved]
In the related art, a low-frequency electric stimulation device cannot be used in water or in a state where skin is wet, because electroconductive electrodes are used. The low-frequency electric stimulation device necessarily lacks a waterproof specification. [Means to Solve the Problem] By a waterproof case of the present invention comprising insulator electrodes for low-frequency electric stimulation and a terminal connection part, the low-frequency electric stimulation device can be used when bathing.. By a waterproof bag of the present invention comprising an insulator electrode film or an insulator electrode sheet for low-frequency electric stimulation having an electrostatic capacitance of 0.36 nF/cm$^2$ or greater, a cordless low-frequency electric stimulation device to which an energization permission sensor function and an underwater communication function are added can be used even in water or when bathing. A low-frequency electric stimulation device having a wireless charging function, an energization permission sensor function, an underwater communication function and the like is configured to have a waterproof structure, thereby implementing a waterproof-type low-frequency electric stimulation device. By dispersing fine particles of an inorganic oxide having a large relative permittivity by 10 to 70 vol% in a resin, a flexible insulator electrode film and an insulator electrode for low-frequency electric stimulation are implemented.

FIG. 1

**Description**

BACKGROUND

1. TECHNICAL FIELD

The present invention relates to technology that enables an electrode to contact skin to safely apply low-frequency electric stimulation to a human body in water.

2. RELATED ART

In the related art, as a low-frequency electric stimulation device, a low-frequency treatment device, an EMS (electric muscle stimulation) device and the like are known. The low-frequency treatment device applies low-frequency electric stimulation to a human body, thereby realizing effects such as aching pain relief, blood circulation promotion, massage effect, improvement on peripheral nerve paralysis, muscle fatigue recovery, and the like. Also, the EMS device applies low-frequency electric stimulation to muscles for muscle-strengthening. Any of the devices is widely spread. The devices each have two or more electroconductive electrodes, and bring the electrodes into contact with a diseased site of the human body to apply low-frequency electric stimulation to the human body. The low-frequency electric stimulation device has electrode cords extending from a device main body, and the electroconductive electrodes are attached to ends of the cords. In the meantime, recently, a cordless low-frequency electric stimulation device where a low-frequency electric stimulation device main body and electroconductive electrodes are integrated into a sheet shape is also spread.
Patent Document 1 discloses an example of the low-frequency electric stimulation device. The low-frequency electric stimulation device comprises a device main body for outputting low-frequency electric stimulation, and corded electroconductive electrodes connected to the device main body. A surface of the device main body is provided with a display unit, an operation unit, a power supply switch, and the like. A user can select an electric stimulation output, an electric stimulation waveform and the like on the operation unit. Since an electrode cord is attached to the electroconductive electrode, the electrode can be contacted to a free position of the human body. Patent Document 2 discloses an example of the cordless low-frequency electric stimulation device. The cordless low-frequency electric stimulation device comprises a main body unit for outputting low-frequency electric stimulation, and a sheet-like electroconductive electrode unit extending leftward and rightward from the main body unit, which are integrally formed. A surface of the main body unit is provided with a push switch-type operation switch, and a user can operate the present device by pushing the push switch-type operation switch. When the present device is attached at a position such as a back that is out of reach, it is impossible to push the operation switch. Therefore, some devices are configured to be wirelessly operated from a smartphone or the like. Since arrangement of the electroconductive electrodes is fixed, an interval between the electrodes cannot be changed. However, the electrodes are small and thin and can be thus easily attached.
Patent Document 3 discloses an example of an electric bathtub where low-frequency stimulation can be obtained in the bathtub. In the electric bathtub, a stainless steel electrode plate is fixed to a bathtub wall, and low-frequency stimulation is transmitted to the electrode from a power supply equipped in a dry separate room. A surface of the electrode is covered with a resin plate having holes so that a bather does not contact the electrode. Patent Document 4 discloses an electroconductive electrode that comes into contact with skin in warm bath water. The electroconductive electrode is provided with an insulating handle part, and a user brings an electroconductive electrode unit into contact with a diseased site with gripping the handle part so as to prevent an electric shock.
Patent Document 5 discloses an example where ceramic insulator electrodes having a large relative permittivity are brought into contact with a head of the human body. A high-frequency alternating current of 50 kH to 500 kHz is applied between the insulator electrodes to lower impedances of the insulator electrodes, and an alternating electric field is applied to tumor cells existing between the electrodes to destroy the tumor cells. However, there is no disclosure about an insulator electrode for low-frequency electric stimulation. Non-Patent Document 1 explicitly states that, when an insulator electrode is considered as an electrode for stimulating a living body, an impedance of the insulator electrode at a frequency of a stimulation signal should be smaller than an impedance of a living tissue and an electrostatic capacitance of several $\mu F/cm^2$ or greater should be secured.

CITATION LIST

PATENT DOCUMENT

Patent Document 1: Japanese Patent Application Publication No. 2009-136472

Patent Document 2: Japanese Patent Application Publication No. H07-136286

Patent Document 3: Japanese Patent No. 4,057,409

Patent Document4: Japanese Utility Model Application Notice No. 12367

Patent Document 5: U.S. Patent No. 8,170,684

NON-PATENT DOCUMENT

Non-Patent Document 1: Medical Electronics and Biological Engineering, Volume 11, Issue 3, Page 161 (June, 1973)

SUMMARY OF INVENTION

TECHNICAL PROBLEM

In the related art, the low-frequency electric stimulation device uses the electroconductive electrodes, and is prevented from being used when the electrode surfaces are wet. When the electroconductive electrodes are used in a state where skin is wet, burn injury or intense irritation pain may be caused due to current being concentrated on sweat glands and pores, which are low resistance portions of the skin. Also, when used for a long time, pH of water changes due to electrolysis of water, which may cause skin inflammation. Also, there is a concern about elution of the electrode material. Therefore, the low-frequency electric stimulation device is used in dry situations such as a room, and necessarily lacks a waterproof specification.

In the electric bathtub, since the electroconductive electrode is fixed to the bathtub wall, the electrode cannot be freely attached to the diseased site. Also, since the device main body is equipped in the separate room, the bather cannot freely adjust the electric stimulation intensity and the electric stimulation waveform. The low-frequency electric stimulation device that is spread in the world and is used in indoor environments applies a pulse voltage between the electroconductive electrodes to cause minute current to flow through the human body. When the electrodes are detached from the skin, an energization circuit is opened, so that current becomes zero. When the low-frequency electric stimulation device detects the zero current, an electric stimulation output is turned off. This is a safety measure for preventing a situation where, if strong electric stimulation is continuously output even when a user stops the use of the device, a person who touches the electrodes without knowing the same is suddenly applied with the strong electric stimulation, and is also to prevent useless power consumption.

However, when the insulator electrodes for low-frequency electric stimulation are used in water, even when the use of the device is stopped and the electrodes are detached from the skin, a circuit is formed through surrounding water, so that current continues to flow. Therefore, the low-frequency electric stimulation device cannot detect that the skin is detached from the electrodes.

The skin is an insulator film having an electrostatic capacitance of about 10 to 20 $nF/cm^2$. Therefore, it has been believed that, in a case where the insulator film is used as an electrode, a combined electrostatic capacitance becomes small unless the film has an electrostatic capacitance significantly larger than the electrostatic capacitance of the skin, and consequently, effective low-frequency electric stimulation cannot be applied to the human body. If a film thickness is reduced so as to increase the electrostatic capacitance of the insulator film, strength of the insulator film is lowered. In order to increase the electrostatic capacitance while increasing the film thickness, an insulator having a large relative permittivity is required. A sintered film formed of ferroelectric ceramic represented by barium titanate has a relative permittivity greater than 15,000 but is broken when bent. A resin is flexible but the relative permittivity thereof is as small as about 3 to 10.

SOLVING MEANS

The invention of waterproof case in accordance with Claim 1 of the present invention is a waterproof case for a non-waterproof-type low-frequency electric stimulation device comprising at least a pair of insulator electrodes for low-frequency electric stimulation; and a terminal connection part for electrically connecting mutually the electrodes and electric stimulation output lines of a non-waterproof-type low-frequency electric stimulation device accommodated in the waterproof case.

The invention of a waterproof bag in accordance with Claim 2 is a waterproof bag for a push switch-type low-frequency electric stimulation device, wherein the bag has a freely openable/closable waterproof seal part, at least one opening is formed in a surface of the bag, and the opening is shielded with an insulator electrode film or an insulator electrode sheet having an electrostatic capacitance of 0.36 nF/cm$^2$ or greater.

The invention of waterproof-type low-frequency electric stimulation device in accordance with Claim 3 is a waterproof-type low-frequency electric stimulation device comprising at least a pair of insulator electrodes for low-frequency electric stimulation; and at least one energization permission switch or energization permission sensor.

The invention of an insulator electrode film for low-frequency electric stimulation in accordance with Claim 4 is an insulator electrode film for low-frequency electric stimulation having an electrostatic capacitance of 0.36 nF/cm$^2$ or greater, wherein the insulator electrode film has fine particles of an inorganic oxide having a large relative permittivity and dispersed by 10 to 70 vol% in a resin. The invention of an insulator electrode film for low-frequency electric stimulation in accordance with Claim 5 is an insulator electrode film for low-frequency electric stimulation according to Claim 4, wherein the resin is a resin of one kind selected from polyvinylidene fluoride (PVDF) monopolymer, polyvinylidene fluoride (PVDF)-based copolymer and polyvinylidene fluoride (PVDF)-based terpolymer or a resin consisting of a mixture of two or more kinds selected from the monopolymer, the copolymer and the terpolymer.

The invention of an insulator electrode for low-frequency electric stimulation in accordance with Claim 6 is an insulator electrode for low-frequency electric stimulation comprising the insulator electrode film for low-frequency electric stimulation according to Claim 4 or 5; an electroconductive substrate formed on a backside of the insulator electrode film; an electric stimulation output core wire electrically connected to the electroconductive substrate; a waterproof cord for covering the electric stimulation output core wire; and an insulating covering for covering exposed live parts of the electroconductive substrate and the electric stimulation output core wire and portions of the insulator electrode film and the waterproof cord.

EFFECTS OF THE INVENTION

By accommodating a main body part of the low-frequency electric stimulation device in the waterproof case for a non-waterproof-type low-frequency electric stimulation device of the invention in accordance with Claim 1, it is possible to safely apply low-frequency electric stimulation to the human body even in water or in a bathtub. By accommodating a waterproof bag compatible type cordless low-frequency electric stimulation device in which an energization permission sensor function and an underwater communication function are added to a cordless low-frequency electric stimulation device in the waterproof bag for a push switch-type low-frequency electric stimulation device of the invention in accordance with Claim 2, it is possible to safely apply low-frequency electric stimulation to the human body even in water or in warm bath water. The waterproof-type low-frequency electric stimulation device of the invention in accordance with Claim 3 allows the low-frequency electric stimulation device or the cordless low-frequency electric stimulation device to safely apply low-frequency electric stimulation to the human body in water, in warm bath water or when bathing.

The invention in accordance with Claims 4 to 6 enables implementation of the flexible insulator electrode for low-frequency electric stimulation that can be used even in water or in the warm bath water. The insulator electrode for low-frequency electric stimulation of the present invention can be used even indoors by placing a wet cloth or the like on a surface of the electrode. In this case, since an adhesive gel is not required, it is possible to solve sticky discomfort and replacement cost of the adhesive gel. In the meantime, the insulator electrode can also be used with the adhesive gel being placed on the surface of the insulator electrode. When the surface of the insulator electrode dries, low-frequency electric stimulation is not transmitted and the human body feels nothing.

BRIEF DESCRIPTION OF THE DRAWINGS

FIG. 1 shows a waterproof case for a non-waterproof-type low-frequency electric stimulation device (overall view).

FIG. 2 shows the waterproof case for a non-waterproof-type low-frequency electric stimulation device (inside).

FIG. 3 shows an insulator electrode for low-frequency electric stimulation.

FIG. 4 shows an energization permission switch and an energization permission sensor.

FIG. 5 shows a terminal connection part and the energization permission switch.

FIG. 6 shows a switch circuit unit of the energization permission sensor.

FIG. 7 shows an energization permission sensor integrated type insulator electrode.

FIG. 8 shows a waterproof case (film window) for a non-waterproof-type low-frequency electric stimulation device.

FIG. 9 shows the waterproof case (film window/inside) for a non-waterproof-type low-frequency electric stimulation device.

FIG. 10 shows a waterproof case (resin sheet case) for a non-waterproof-type low-frequency electric stimulation device.

FIG. 11 shows a waterproof-type low-frequency electric stimulation device (embodiment 1).

FIG. 12 is a circuit block diagram of the waterproof-type low-frequency electric stimulation device (embodiment 1) (energization permission switch).

FIG. 13 is a circuit block diagram of the waterproof-type low-frequency electric stimulation device (embodiment 1) (energization permission sensor).

FIG. 14 shows a waterproof bag for a push switch-type low-frequency electric stimulation device (operation surface-side).

FIG. 15 shows the waterproof bag for a push switch-type low-frequency electric stimulation device (electrode surface-side).

FIG. 16 shows the waterproof bag for a push switch-type low-frequency electric stimulation device (sectional view).

FIG. 17 shows an insulator electrode sheet.

FIG. 18 shows a waterproof bag compatible type cordless low-frequency electric stimulation device (operation surface-side).

FIG. 19 shows the waterproof bag compatible type cordless low-frequency electric stimulation device (electrode surface-side).

FIG. 20 is a circuit block diagram of the waterproof bag compatible type cordless low-frequency electric stimulation device (energization permission sensor).

FIG. 21 shows an energization permission sensor for a waterproof bag compatible type cordless low-frequency electric stimulation device.

FIG. 22 shows a waterproof-type low-frequency electric stimulation device (embodiment 3) (operation surface-side).

FIG. 23 shows the waterproof-type low-frequency electric stimulation device (embodiment 3) (electrode surface-side).

FIG. 24 shows the waterproof-type low-frequency electric stimulation device (embodiment 3) (sectional view).

FIG. 25 shows a fixing belt attached to a waterproof bag for a push switch-type low-frequency electric stimulation device.

FIG. 26 shows an extendible fixing belt of a separate body.

FIG. 27 shows a waterproof-type low-frequency electric stimulation device (embodiment 4) (fixing belt integrated type).

FIG. 28 shows a waterproof-type low-frequency electric stimulation device (embodiment 5) (shoulder-mounted integrated type).

FIG. 29 shows a relation between an electrostatic capacitance per a unit area in which a human body feels low-frequency electric stimulation and a peak voltage (overall).

FIG. 30 shows the relation between the electrostatic capacitance per a unit area in which the human body feels low-frequency electric stimulation and the peak voltage (vertical axis is enlarged).

FIG. 31 shows the relation between the electrostatic capacitance per a unit area in which the human body feels low-frequency electric stimulation and the peak voltage (horizontal axis is enlarged).

DESCRIPTION OF EXEMPLARY EMBODIMENTS

<Embodiment of Waterproof Case for Non-Waterproof-Type Low-Frequency Electric Stimulation Device>

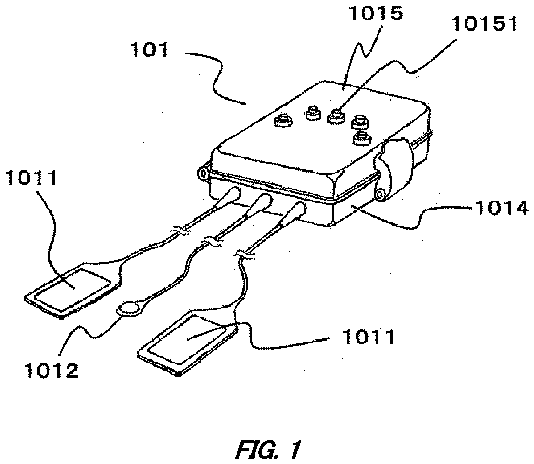

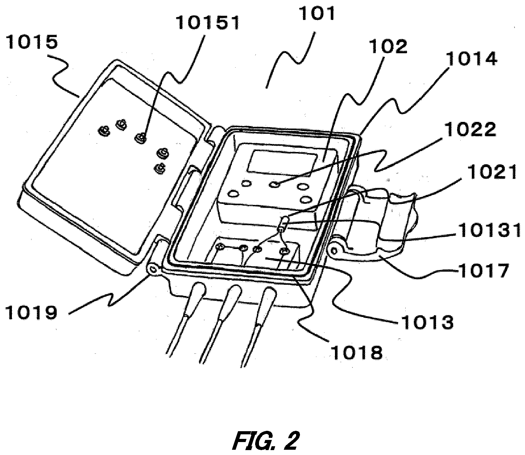

A waterproof case for a non-waterproof-type low-frequency electric stimulation device is a case that enables a main body of a non-waterproof-type low-frequency electric stimulation device for indoor use to be used while bathing by accommodating the main body of the non-waterproof low-frequency electric stimulation device in the case. FIG. 1 shows an entire configuration of a waterproof case 101 for a non-waterproof-type low-frequency electric stimulation device according to the present invention. FIG. 2 shows an internal configuration of the waterproof case 101 with an upper lid 1015 being opened. The present waterproof case 101 comprises an upper lid 1015, a lower case 1014, insulator electrodes 1011 for low-frequency electric stimulation and an energization permission switch 1012 connected to the lower case, and a terminal connection part 1013 for electrically connecting electric stimulation output lines from an accommodated non-waterproof-type low-frequency electric stimulation device main body 102 and the insulator electrodes for low-frequency electric stimulation and the energization permission switch. The number of the present insulator electrodes may be two or more and the number is not limited.
When using the present waterproof case 101, it can be used by inserting a connection plug 10131 extending from the terminal connection part 1013 into an output jack part 1021 of the non-waterproof-type low-frequency electric stimulation device main body 102 to be accommodated, accommodating the device main body 102 in the present waterproof case 101, closing the upper lid 1015 with an O-ring 1018 being sandwiched therebetween, and fastening a buckle 1017. The upper lid 1015 is provided with waterproof switches 10151, and the operation switches 1022 of the present device main body 102 can be operated by pushing the waterproof switches. As a material of the upper lid 1015 and the lower case 1014 of the present waterproof case, general resin materials such as acrylic resin, polycarbonate resin, vinyl chloride resin, ABS resin and the like can be used. In the present embodiment, the upper lid 1015 and the lower case 1014 are coupled by a hinge 1019 and are sealed by the O-ring 1018 sandwiched therebetween. However, the structure of the waterproof case does not need to be limited thereto. The present waterproof case is used while being hung on a wall of a bathroom. Alternatively, the present waterproof case may be used while being floated on the warm water surface. Since the waterproof case main body is not submerged in water, a simple waterproof structure can be taken. A spacer, an engaging piece and the like may be arranged as appropriate so that the accommodated non-waterproof-type low-frequency electric stimulation device main body 102 does not move in the waterproof case. By setting an electrostatic capacitance of the insulator electrode as appropriate, it is possible to cope with a non-waterproof-type low-frequency electric stimulation device main body having diverse output voltage specifications.

[Insulator Electrode 1011 For Low-Frequency Electric Stimulation]

(Operating Principle and Required Electrostatic Capacitance and Relative Permittivity)

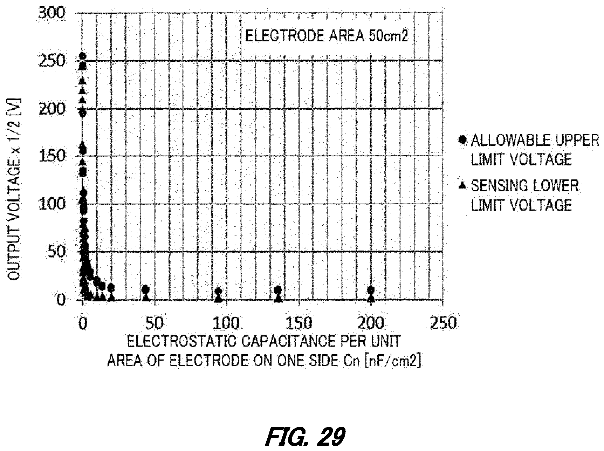

An insulator film used as an electrode is referred to as an insulator electrode film. Also, the electrode for which the insulator electrode film is used is referred to as an insulator electrode. The present inventor measured a relation between an electrostatic capacitance of an insulator electrode for low-frequency electric stimulation and a peak voltage of a low-frequency electric stimulation rectangular wave pulse, which allows a human body to feel low-frequency electric stimulation in water of about 40°C. As the insulator electrode film used for measurement, a barium titanate-based sintered film and diverse resin films were used. For low-frequency electric stimulation, a frequency of about 1 Hz to 1200 Hz is generally used. However, since frequency dependency of the peak voltage at which the human body feels low-frequency electric stimulation is small, 50 Hz was used as a standard frequency. Since an on-time period of the rectangular wave pulse voltage is generally tens of $\mu$ seconds to hundreds of $\mu$ seconds, 300 $\mu$sec was set. A cycle in which 200 rectangular wave pulses of the same polarity were generated, and after a pause of 0.5 second, 200 rectangular wave pulses of the opposite polarity were generated was repeated. The larger an area of the insulator electrode is, the lower the peak voltage becomes at which the human body feels low-frequency electric stimulation. However, since the peak voltage at which the human body starts to feel low-frequency electric stimulation becomes constant when the area exceeds 50 $cm^2$, the area of the insulator electrode was set to 50 $cm^2$.
A measurement result is shown in FIG. 29. The vertical axis indicates a voltage [V] that is half of the voltage of the peak voltage and is applied to the insulator electrode on one side. The horizontal axis indicates an electrostatic

capacitance Cn [nF/cm$^2$] per unit area of the insulator electrode on one side for low-frequency electric stimulation. The black triangular mark (▲) indicates a sensing lower limit voltage at which the human body starts to feel low-frequency electric stimulation. The black circular mark (●) indicates an allowable upper limit voltage at which low-frequency electric stimulation is so intensive that the human body starts to feel discomfort or pain. It can be seen that when Cn increases, both the voltages level off, and when Cn decreases, both the voltages rise sharply. Since the electrostatic capacitance of skin in water is about 20 nF/cm$^2$, it can be seen that when Cn of the insulator electrode becomes greater than 20 nF/cm$^2$, a rate-controlling effect due to serial combination of the electrostatic capacitances disappears. Also, it was clarified that there is a condition under which even an insulator electrode having an electrostatic capacitance significantly smaller than the electrostatic capacitance of skin can apply low-frequency electric stimulation to the human body.

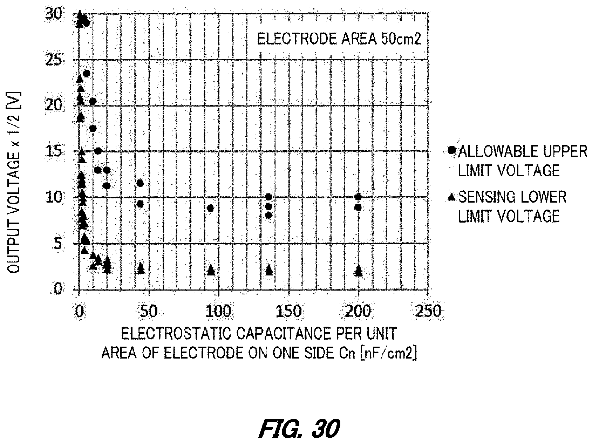

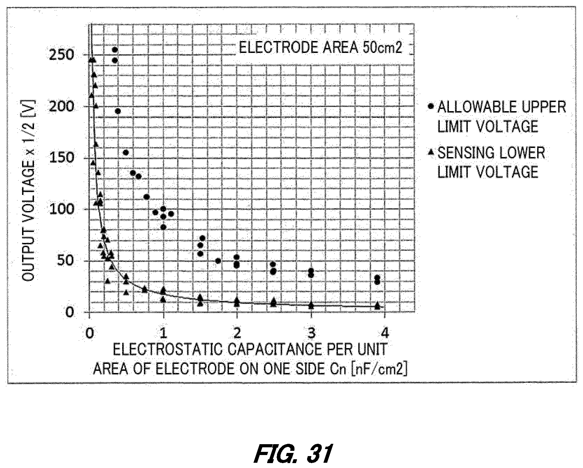

FIG. 30 is an enlarged view of the vicinity of the origin of the vertical axis of FIG. 29. At 44 nF/cm$^2$ or greater, the sensing lower limit voltage is constant at about 2 V. On the other hand, at 94 nF/cm$^2$ or greater, the allowable upper limit voltage is constant at about 9 V. That is, it is not effective to increase Cn beyond 94 nF/cm$^2$, from a standpoint of lowering the allowable upper limit voltage. FIG. 31 is an enlarged view of the vicinity of the origin of the horizontal axis of FIG. 29. Both the sensing lower limit voltage and the allowable upper limit voltage rise sharply as Cn becomes smaller.

With respect to the sensing lower limit voltage (U[V]), a power approximation curve was obtained by the least square method for data of five subjects in a range where Cn is smaller than 4 nF/cm$^2$. As a result, an equation (1) was obtained.

$$U = 17.7 * Cn^{\wedge} \cdot 0.8611) \qquad (1)$$

$$(R^{\wedge}2 = 0.9551)$$

If Cn is gradually made smaller, the sensing lower limit voltage rises sharply, which is not desirable from standpoints of safety and effectiveness. Therefore, a practical maximum value of U is considered to be 250 V. By using the equation (1) to determine Cn at U=250V, Cn=0.063 nF/cm$^2$ is obtained. This value can be said to be the minimum electrostatic capacitance per unit area of insulator electrode on one side, which is necessary to apply electric stimulation to the human body by using low-frequency pulse voltage. On the other hand, the allowable upper limit voltage is 255 V when Cn is 0.36 nF/cm$^2$. Therefore, the minimum Cn necessary to feel stimulation up to the allowable upper limit is considered to be 0.36 nF/cm$^2$. In this case, the peak value of 510 V is applied between the electrodes and the peak current reaches 2.3 A. However, the time constant is about 1 psec, and the human body will not be injured.

From the above results, it can be concluded that in the insulator electrode for low-frequency electric stimulation for applying low-frequency electric stimulation to the human body, electrostatic capacitance of 0.063 nF/cm$^2$ or greater per unit area of insulator electrode on one side is required to apply sensing lower limit stimulation, and further, electrostatic capacitance of 0.36 nF/cm$^2$ or greater is required to apply allowable upper limit stimulation. Also, it can be concluded that as the output voltage (a voltage to be applied to the insulator electrodes on both sides) of the low-frequency electric stimulation device, a peak voltage of 4 V or higher is required to apply sensing lower limit stimulation and a peak voltage of 18 V or higher is required to apply allowable upper limit stimulation.

From the above results, the electrostatic capacitance of the insulator electrode for low-frequency electric stimulation is desirably 0.36 nF/cm$^2$ or greater. More desirably, the electrostatic capacitance is 1 nF/cm$^2$ or greater. When the electrostatic capacitance is 1 nF/cm$^2$ or greater, it is possible to apply the allowable upper limit stimulation at a pulse peak value of 200 V that is the maximum value used in a household low-frequency treatment device. More desirably, the electrostatic capacitance is 2 nF/cm$^2$ or greater. When the electrostatic capacitance is 2 nF/cm$^2$ or greater, it is possible to apply the allowable upper limit stimulation at a pulse peak value of 100 V that is used in household low-frequency treatment devices that are commonly used. Most desirably, the electrostatic capacitance is 5 nF/cm$^2$, such that human body can feel the allowable upper limit stimulation even at a safety extra low voltage (SELV).

(Insulator Electrode Film 10111 For Low-Frequency Electric Stimulation)

The electrostatic capacitance C[F] of the insulator electrode film is given by the following equation.

$$C = \varepsilon_0 \varepsilon_r \times S/d \qquad (2)$$

Herein, $\varepsilon_0$ is vacuum permittivity 8.854 × 10$^{-12}$[F/m], $\varepsilon_r$ is relative permittivity of the insulator electrode film, S is an area [m$^2$] of the electrode, and d is a thickness [m] of the insulator electrode film. From the equation, the electrostatic

capacitance Cn [nF/cm$^2$] per area 1 cm$^2$ of the insulator electrode can be conveniently determined by the following equation.

$$Cn[nF/cm^2] = 0.8854\varepsilon_r/t \qquad (3)$$

Here, t is a thickness [$\mu$m] of the insulator electrode film. That is, for example, when the relative permittivity is 10 and the film thickness is 10 $\mu$m, Cn is 0.8854 nF/cm$^2$.

Therefore, it is desirable that the insulator electrode film has a greater relative permittivity. The barium titanate-based ferroelectric ceramic has a relative permittivity greater than 15,000 but is broken when bent. The insulator electrode for low-frequency electric stimulation is preferably flexible because it is used in contact with the human body. A resin film is flexible but its relative permittivity is as small as about 3 to 10. Therefore, electrostatic capacitance cannot be obtained unless it is made thin. However, if the resin film is made thin, mechanical strength, withstand voltage and durability are lowered. If the film thickness is increased, mechanical strength, withstand voltage and durability are increased but the electrostatic capacitance is reduced, and consequently, it cannot be used unless the applied voltage is increased. In this way, the film thickness and the electrostatic capacitance have a tradeoff relation.

Table 1 shows the relative permittivity of the insulator electrode film that is necessary to achieve the required electrostatic capacitance Cn [nF/cm$^2$]. The film thickness of the resin film is set to 4 $\mu$m or greater. If the film thickness is smaller than 4 $\mu$m, the film is too thin, which makes the film impractical due to poor mechanical strength, withstand voltage and durability. For example, in a case where Cn is 0.36, the peak voltage of 500V is required. However, in this case, the thickness of 4 $\mu$m is insufficient in terms of withstand voltage. When the film thickness is 20 $\mu$m or greater, there is no problems in terms of withstand voltage and durability. Therefore, resin films having relative permittivity of 8.1 or greater can be used. For example, it can be seen that polyvinylidene fluoride (PVDF) can be used. 500 V is the peak voltage that is applied to the insulator electrodes on both sides. However, it is desirable to have withstand voltage of 500 V and durability with an insulator electrode on one side only. In a case where Cn is 1, the peak voltage is 200 V. However, since 10 $\mu$m is required in terms of withstand voltage and durability, a relative permittivity of 11.3 or greater is required. In a case where Cn is 2, the peak voltage is 100 V. Even a thickness of 4 $\mu$m can withstand in terms of withstand voltage but there is concern about durability. The thickness is desirably 10 $\mu$m or greater. That is, the relative permittivity is preferably 22.6 or greater. In a case where Cn is 5, the film can be used at an applied voltage of 42 V. In this case, even a film thickness of 4 $\mu$m can be used because the applied voltage is low, and the relative permittivity is preferably 22.6 or greater.

The applied voltage is desirably low, and the film thickness is desirably thick. That is, it is desirable to use the film in a lower right direction of Table 1. That is, the relative permittivity is desirably greater. As described above, the relative permittivity needs to be 8 or greater, and is desirably a value of 25 or greater. More desirably, the relative permittivity is a value of 50 or greater. Most desirably, the relative permittivity is a value of 100 or greater.

Table 1. Relative permittivity of insulator electrode film for achieving the required electrostatic capacitance

[Table 1]

| | | REQUIRED ELECTROSTATIC CAPACITANCE Cn[nF/cm2] | | | |
|---|---|---|---|---|---|
| | | 0.36 | 1 | 2 | 5 |
| | | APPLY 500V | APPLY 200V | APPLY 100V | APPLY 42V |
| | | | | | |
| FILM THICKNESS | 4$\mu$m | 1.6 | 4.5 | 9.0 | 22.6 |
| | 10$\mu$m | 4.1 | 11.3 | 22.6 | 56.5 |
| | 20$\mu$m | 8.1 | 22.6 | 45.2 | 112.9 |

As the material of the insulator electrode film, polyvinylidene fluoride (PVDF) monomer, PVDF-based materials such as copolymer of vinylidene fluoride (VDF) and chlorotrifluoroethylene (CTFE), chlorofluoroethylene (CFE), hexafluoropropylene (HFP), chlorodifluoroethylene (CDFE), trifluoroethylene (TrFE) and the like or polyvinylidene fluoride (PVDF)-based terpolymer such as P(VDF-TrFE-CTFE), P(VDF-TrFE-CFE), P(VDF-TrFE-HFP), P(VDF-TrFE-CDFE), P(VDF-TFE-CTFE), P(VDF-TFE-CFE), P(VDF-TFE-HFP), P(VDF-TFE-CDFE) and the like may be exemplified. Herein, TFE represents tetrafluoroethylene. Also, a mixture of a PVDF homopolymer and the above-mentioned copolymer or terpolymer, or a mixture of the above-mentioned copolymer and terpolymer may be exemplified. Further, a film in which the above-mentioned copolymer or terpolymer, or a mixture of the above-mentioned copolymer, terpolymer and PVDF homopolymer is extended in a uniaxial or biaxial direction is also possible. In particular, P(VDF-TrFE-CTFE) terpolymer or P(VDF-TrFE-CFE) terpolymer can be favorably used because a relative permittivity of about 40 to 50 can be obtained at 40°C.

It is possible to significantly improve relative permittivity of a resin by dispersing fine particles of an inorganic oxide having a large relative permittivity in the resin. As the resin, in addition to the above-mentioned PVDF-based resin, an epoxy resin, a polyimide resin, an acrylic resin, a mixed resin of aromatic polyimide and bismaleimide, and the like can be used. Also, as the fine particles of an inorganic oxide having a large relative permittivity, barium titanate, CCTO($CaCu_3Ti_4O_{12}$), BLTC in which barium and titanium of barium titanate are each replaced with lanthanum and chromium, NiO(LTNO) in which lithium and titanium are co-doped, strontium titanate and the like may be exemplified. In particular, barium titanate is preferable. For the purpose of increasing the relative permittivity of a composite film, a large relative permittivity can be achieved by dispersing carbon black, carbon nanofibers, silver nanowires and the like. However, when such electroconductive fillers are dispersed, the electrochemical non-reactivity and dielectric strength in water are lowered, which is not desirable.

It is possible to obtain a favorable film by dispersing fine particles of barium titanate in a range of 10 vol% to 70 vol% in PVDF or epoxy resin. In a range of 10 vol% or less, the improvement on the relative permittivity is small, and in a range of 70 vol% or more, mechanical strength and dielectric strength as the resin are impaired. For example, a film in which fine particles of barium titanate (BT-HP150 available from KCM Corporation) are dispersed by 35 vol% in a two-part flexible epoxy resin (Bond E2420) has a relative permittivity of about 40 at 40°C. Therefore, an electrostatic capacitance of about 2.1 nF/cm$^2$ can be achieved by using a film having a thickness of 17 $\mu$m.

As a favorable embodiment, a measurement result for the insulator electrode film in which fine particles of barium titanate (BT-HP150 available from KCM Corporation) are dispersed by 30 vol% in P(VDF-TrFE-CTFE) terpolymer made of PIEZOTECH (registered trademark) available from ARKEMA Corporation is shown in Table 2. An insulator electrode film having a film thickness of 11 $\mu$m and a size of 80 × 110 mm was formed on a stainless foil having a thickness of 20 $\mu$m by solvent casting. The electrode area$\Phi$ was 15 mm, and the electrostatic capacitance and the dielectric loss (tan$\delta$) were measured at 38°C by using an LCR meter (HP4262A). As a result, the electrostatic capacitance at 1 kHz was 10.1 nF/cm$^2$, the dielectric loss (tan$\delta$) was 0.05, and the relative permittivity was 103.

When the insulator film is immersed in water and used as an electrode for low-frequency electric stimulation, due to polarization in the insulator film, displacement current flows but electron conduction current does not flow, and transfer of electrons between the electrode and water does not substantially occur. Therefore, electrochemical reactions such as electrolysis and elution of electrode materials do not occur. Also, even when the insulator electrode film is directly brought into contact with wet skin, current is not concentrated on low resistance portions such as sweat glands and pores. Similarly, even when a part of the electrode separates from the skin, current is not concentrated on a part in contact with the skin. Also, even when DC current is leaked to the electrode unit due to a circuit accident, the insulator film blocks the DC current. In this way, it is possible to safely apply electric stimulation to the human body even in water by using the insulator electrodes.

Table 2. Characteristics of insulator electrode film in which fine particles of barium titanate are dispersed in P(VDF-TrFE-CTFE) terpolymer

[Table 2]

INSULATOR
ELECTRODE FILM 11µmt、80×110mm

STAINLESS FOIL (20µm)

【MEASUREMENT CONDITIONS】

FILM THICKNESS: 11µm
MEASUREMENT TEMPERATURE: 38°C
MEASUREMENT AREA: Φ15mm
MEASURING INSTRUMENT: HP 4262A

| | Cn[nF/cm²] | RELATIVE PERMITTIVITY | tan δ |
|---|---|---|---|
| 120Hz | 10.7 | 109 | 0.04 |
| 1KHz | 10.1 | 103 | 0.05 |
| 10KHz | 9.3 | 94 | 0.17 |

(Configuration of Insulator Electrode 1011 For Low-Frequency Electric Stimulation)

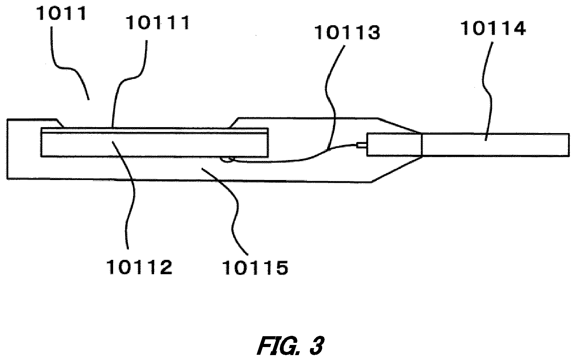

FIG. 3 shows one embodiment of the insulator electrode for low-frequency electric stimulation. The insulator electrode 1011 for low-frequency electric stimulation comprises an insulator electrode film 10111 formed on an electroconductive substrate 10112, an electric stimulation output core wire 10113 electrically connected to the electroconductive substrate 10112, a waterproof cord 10114 for protecting the electric stimulation output core wire, and an insulating covering 10115 for covering the electroconductive live part such as the electroconductive substrate and the electric stimulation output core wire, a part of the insulator electrode film and a part of the waterproof cord.

{Electroconductive Substrate}

For the electroconductive substrate 10112, a metal sheet such as copper, aluminum, stainless steel, titanium, nickel, titanium nickel alloy and the like, a metal mesh with an electroconductive adhesive material, a film of an organic electroconductive ink such as PEDOT/PSS or a film obtained by vapor depositing or sputtering metal such as platinum, aluminum, titanium, nickel or the like can be used. After forming a film of an organic electroconductive ink or metal in vacuum, a metal foil or sheet may be further bonded using an electroconductive adhesive. The insulator electrode film may be formed on the electroconductive substrate or the insulator electrode film may be first formed and the electroconductive substrate may be then formed on a surface thereof.

{Insulating Covering}

The insulating covering 10115 may be formed by insert molding with a thermoplastic resin, potting of a resin, sealing welding with a thermoplastic resin sheet, or the like. Alternatively, the insulating covering 10115 may be configured by a plurality of resin components, and interfaces between the resin components and an interface with a surface of the insulator electrode film may be sealed with an adhesive, a sticking material, a sealant or the like. Also, the insulating covering 10115 may have such a configuration that it can be crimp-coupled by a screw, a press-fitting mechanism or the like with a packing, an O-ring or the like being sandwiched therebetween.

{Waterproof Cord}

For the waterproof cord 10114, an insulated covered electric wire, a vinyl cabtyre cable and the like may be used. Also, as a waterproof protection pipe of the insulated covered electric wire, for example, a silicon tube, a soft fluorine resin tube or the like may be bonded to the insulating covering 10115 and the insulated covered electric wire may be inserted therein. The length of the waterproof cord is about 2m. However, when inserting the insulated covered electric wire into the silicon tube, it is difficult to insert the insulated covered electric wire due to a large sliding resistance of the surface. In a case where the fluorine covered electric wire and/or the soft fluorine resin tube is used, the sliding resistance is small and it is easy to insert the insulated covered electric wire, so it is not necessary to increase an inner diameter of the tube beyond necessity, which is favorable.

The insulator electrode for low-frequency electric stimulation of the present invention can be used not only in water but also indoors. In a case where the insulator electrode is used indoors, it may be used with a water-absorbing cloth containing water or an electroconductive adhesive pad arranged on the surface of the insulator electrode. When the water-absorbing cloth is used, both the water-absorbing cloth and the present electrode can be washed with water and can be used hygienically. Also, it is not necessary to replace and discard consumables such as the electroconductive adhesive pad. When the surface of the insulator electrode is dry, an electric field is blocked by an air layer between the insulator electrode film and the skin, and consequently no electric stimulation is felt.

[Energization Permission Switch 1012]

In a case where the insulator electrode for low-frequency electric stimulation is used in water, since current continues to flow through water even when the electrode is detached from the skin, it is not possible to determine on the low-frequency electric stimulation device main body-side whether the use is over. There is a risk of the electrode being left in water with the intense electric stimulation being output, and if a third party unexpectedly touches the surface of the electrode without knowing it, they may receive a strong shock. Also, electric power is wasted. In order to prevent such accidents, it is necessary to provide a mechanism for stopping the electric stimulation output when the user stops the use.

The energization permission switch 1012 is a momentary switch that is turned on when a pressing force is applied to a surface of the switch, stays on only while the switch is pressed, and is turned off when the pressing force is released, which is accommodated in a flexible waterproof covering so that it can be used even in water. The pressing may be applied with a hand or a part of the body by the user or may be applied by bonding the switch to a fixing belt together with the insulator electrode and wrapping the belt around the human body. For the energization permission switch, a push switch such as a membrane switch and a tactile switch may be used.

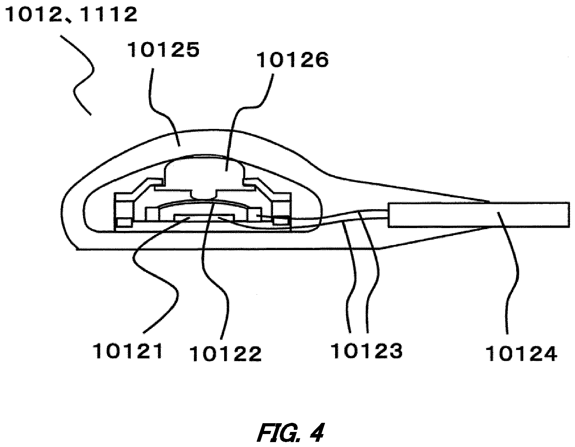

FIG. 4 shows an example of a sectional structure of the energization permission switch 1012. In the example, a tactile switch is covered with the waterproof covering. A reference sign 10121 indicates a lower electrode, and a reference sign 10122 indicates an inversion spring upper electrode made of metal for providing a sense of click. A switch core wire 10123 of a waterproof cord 10124 is electrically connected to each of the electrodes. A reference sign 10126 indicates a plunger for transmitting the pressing force to the inversion spring. The tactile switch is covered with a flexible waterproof covering 10125, and can be turned on when pressed from above. As a material of the waterproof covering 10125, thermoplastic elastomer, thermosetting elastomer, thermoplastic resin and the like are favorable. The waterproof covering may be formed by insert molding, potting, welding of a resin sheet, or the like.

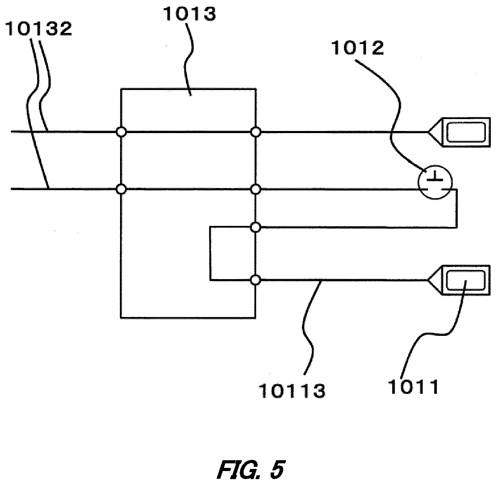

FIG. 5 is a wiring diagram of the energization permission switch 1012. The present energization permission switch is electrically connected in series with an electric stimulation output line 10132 on one side and the electric stimulation output core wire 10113 of the insulator electrode 1011 on one side at the terminal connection part 1013. With such configuration, when pressing force is applied to the energization permission switch 1012, the electric stimulation output can be turned on, and when the pressing force is released, the electric stimulation output can be turned off. In this way, it is possible to block the electric stimulation output when not in use in water.

[Energization Permission Sensor 1112]

When an electric stimulation voltage or current rises, it may not be appropriate to turn on/off the electric stimulation output directly by the momentary switch such as a membrane switch and a tactile switch, due to occurrence of a spark. In this case, the energization permission switch 1012 may be used as a sensor simply for detecting a use state, and a switch capable of turning on/off the electric stimulation output according to an output signal of the sensor may be separately provided. When the energization permission switch 1012 is used as a sensor as described above, it is referred to as an energization permission sensor 1112.

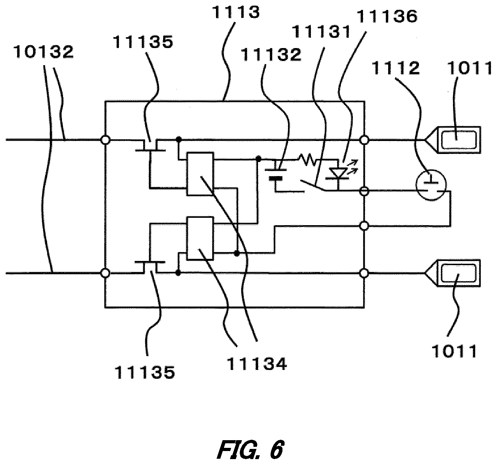

FIG. 6 shows an exemplary embodiment of a sensor circuit 1113 in a case where the energization permission switch 1012 is used as the energization permission sensor 1112. The sensor circuit 1113 is configured by a power supply switch 11131, a battery 11132, a photo-coupler 11134, an N-channel MOSFET 11135, an LED lamp 11136, and the like. By turning the power supply switch 11131 on, the sensor circuit turned on and the LED lamp 11136 is turned on. By including a power supply switch, it is possible to prevent useless consumption of the battery even when the energization permission sensor is erroneously pushed when not in use. The sensor circuit 1113 is accommodated in the terminal connection part 1013.

When the energization permission sensor 1112 is pressed and is turned on, current is supplied to the photo-coupler 11134, so that a photovoltaic voltage from the photo-coupler is applied to a gate of the MOSFET 11135 to turn on the MOSFET 11135 and the electric stimulation output is transmitted to the insulator electrode 1011 for low-frequency electric stimulation. When the momentary switch of the energization permission sensor 1112 is turned off, the current supply to the photo-coupler is turned off, so that the MOSFET is turned off and the electric stimulation output is also cut off. With this configuration, only a drive voltage of the photo-coupler is applied to the momentary switch of the energization permission sensor, so that the burden on the momentary switch is reduced and the durability is improved. Also, the energization permission sensor can be insulated from the electric stimulation output line 10132, so that the safety is improved.

For the switch that is used as the energization permission sensor, a membrane switch, a strain gauge, a pressure sensor and the like may also be used, in addition to the tactile switch. The example of the circuit in which the N-channel MOSFET and the photo-coupler are used as the switch of the electric stimulation output line has been described. However, as the circuit for turning on/off the electric stimulation output by receiving the output of the energization permission sensor, a variety of circuits can be contemplated by one skilled in the art, and the present invention is not necessarily limited to the above circuit.

[Embodiment Where Insulator Electrode For Low-Frequency Electric Stimulation and Energization Permission Sensor Are Integrated]

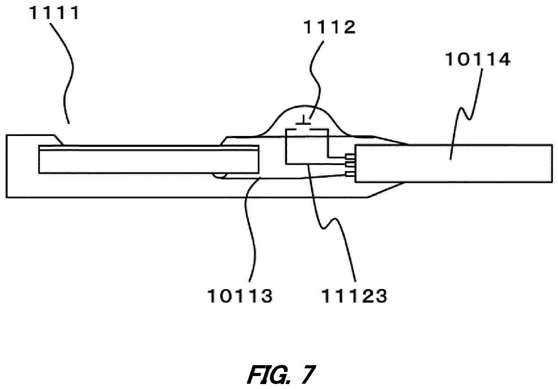

FIG. 7 shows an example of an energization permission sensor integrated type insulator electrode 1111 for low-frequency electric stimulation where the insulator electrode 1011 for low-frequency electric stimulation is integrally provided with the energization permission sensor 1112. The energization permission sensor 1112 is embedded in a surface of the insulator electrode 1011. When the electrode is pressed against the human body, the sensor is turned on, and when the electrode is detached from the human body, the sensor is turned off. Both a sensor core wire 11123 of the sensor and the electric stimulation output core wire 10113 of the electrode pass through the waterproof cord 10114. The energization permission sensor may also be provided on a backside of the electrode. With this configuration, the insulator electrode for low-frequency electric stimulation and the energization permission sensor can be integrated without being separated in the waterproof case for non-waterproof-type low-frequency electric stimulation. In this case, at least one of the plurality of insulator electrodes is preferably the energization permission sensor integrated type insulator electrode 1111 for low-frequency electric stimulation. In a case where the plurality of energization permission sensor integrated type insulator electrodes for low-frequency electric stimulation is used, when any one is turned on, the electric stimulation output is turned on.

<Another Embodiment of Waterproof Case for Non-Waterproof-Type Low-Frequency Electric Stimulation Device>

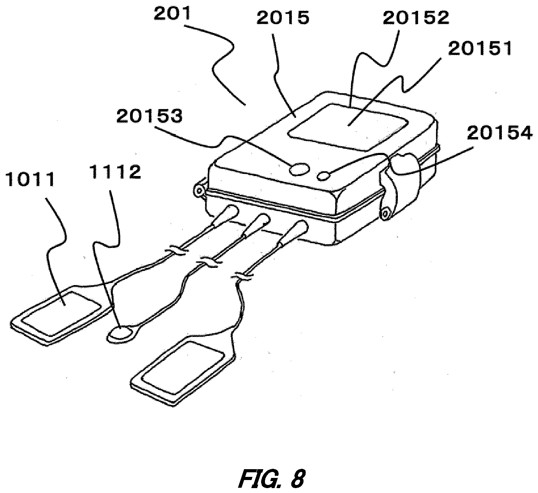

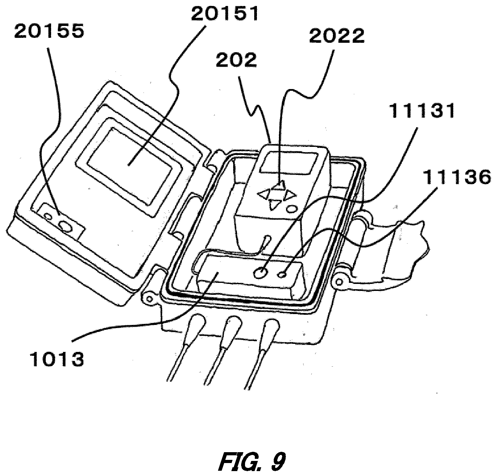

FIG. 8 shows a waterproof case 201 for non-waterproof-type low-frequency electric stimulation device, in which the waterproof switch part of the waterproof case 101 for a non-waterproof-type low-frequency electric stimulation device shown in FIG. 1 is replaced with a transparent resin film. Also, FIG. 9 shows an aspect where an upper lid 2015 of the waterproof case 201 is opened. The upper lid 2015 is formed with an opening 20152, and the opening is shielded by a transparent resin film 20151. Similarly, an opening 20153 for pushing a power supply switch 11131 of the sensor circuit 1113 and an opening 20154 for seeing the LED lamp 11136 are formed, and the openings are shielded by a transparent resin film 20155. An operation switch 2022 of the accommodated non-waterproof-type low-frequency electric stimulation device main body 202 can be operated by pressing the same with a finger from above the transparent resin film 20151. In the present example, the example where the energization permission sensor 1112 is provided is shown. However, the energization permission switch may be provided. When a transparent material is used for the upper lid 2015, the opening 20154 is not required.

As a material of the transparent resin film, a transparent and flexible film is favorable. In particular, a thermoplastic resin having a low water-vapor transmission ratio is desirable. For example, polyvinyl chloride (PVC), polyethylene terephthalate (PET), ethylene-tetrafluoroethylene copolymer (ETFE), polyvinylidene chloride (PVDC), polyethylene (PE), polypropylene (PP), polyvinylidene fluoride (PVDF), tetrafluoroethylene-perfluoroalkyl vinyl ether copolymer (PFA), tetrafluoroethylene-hexafluoropropylene copolymer (FEP), polychlorotrifluoroethylene (PCTFE), polytetrafluoroethylene (PTFE) and the like may be exemplified. The present invention is not limited thereto. The resin film and the upper lid may be bonded by welding, a double-sided tape or the like.

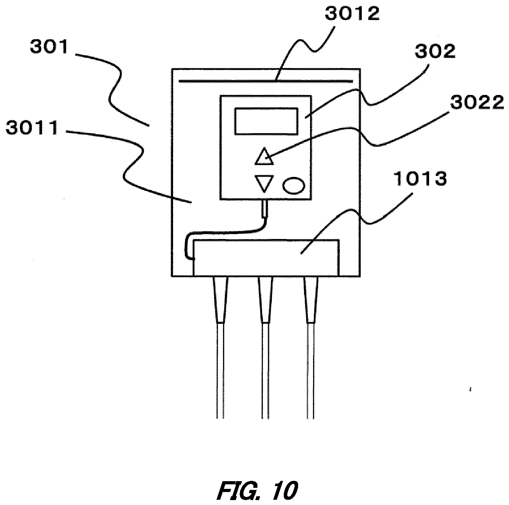

FIG. 10 shows an example where a bag of flexible resin is used as the waterproof case for a non-waterproof-type low-frequency electric stimulation device. A waterproof case 301 for a non-waterproof-type low-frequency electric stimulation device is configured by a bag 3011 made of transparent and flexible resin, and has a freely openable/closable waterproof seal 3012. An operation switch 3022 of the accommodated non-waterproof-type low-frequency electric stimulation device main body 302 can be operated by pressing the same with a finger from above the waterproof case. In the waterproof case, the terminal connection part 1013 is accommodated, and the insulator electrode for low-frequency electric stimulation and the energization permission switch or the energization permission sensor are connected to the terminal connection part.

As a material of the transparent and flexible bag made of resin, a transparent and flexible film is favorable. In particular, a thermoplastic resin having a low water-vapor transmission ratio is desirable. For example, polyvinyl chloride (PVC), polyethylene terephthalate (PET), ethylene-tetrafluoroethylene copolymer (ETFE), polyvinylidene chloride (PVDC), polyethylene (PE), polypropylene (PP), polyvinylidene fluoride (PVDF), tetrafluoroethylene-perfluoroalkyl vinyl ether copolymer (PFA), tetrafluoroethylene-hexafluoropropylene copolymer (FEP), polychlorotrifluoroethylene (PCTFE), polytetrafluoroethylene (PTFE) and the like may be exemplified. The present invention is not limited thereto.

<Embodiment 1 of Waterproof-Type Low-Frequency Electric Stimulation Device>

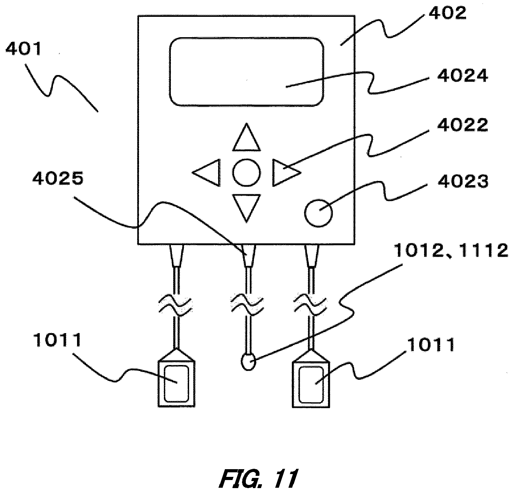

FIG. 11 shows an example of the waterproof-type low-frequency electric stimulation device. A waterproof-type low-frequency electric stimulation device 401 has the insulator electrodes 1011 for low-frequency electric stimulation, and the energization permission switch 1012 or the energization permission sensor 1112 provided to a waterproof-type low-frequency electric stimulation device main body 402. The waterproof-type low-frequency electric stimulation device main body 402 is provided with a power supply switch 4023, an operation switch 4022 capable of selecting an intensity and a waveform pattern of the electric stimulation output, a display unit 4024, a waterproof cord collection part 4025, and the like. The power supply switch and the operation switch are each configured by a membrane switch or a tactile switch having a waterproof structure. For the waterproof cord collection part, a general purpose waterproof cable gland or the like can be used. The device main body 402 has a waterproof structure and can float on a warm water surface. Also, the device main body 402 can be used while being hung on a wall surface. The waterproof structure can be implemented by technology similar to a general waterproof-type electronic device. In the present embodiment, instead of the insulator electrode 1011 for low-frequency electric stimulation, and the energization permission switch 1012 or the energization permission sensor 1112, the energization permission sensor integrated type insulator electrode 1111 for low-frequency electric stimulation may also be used.

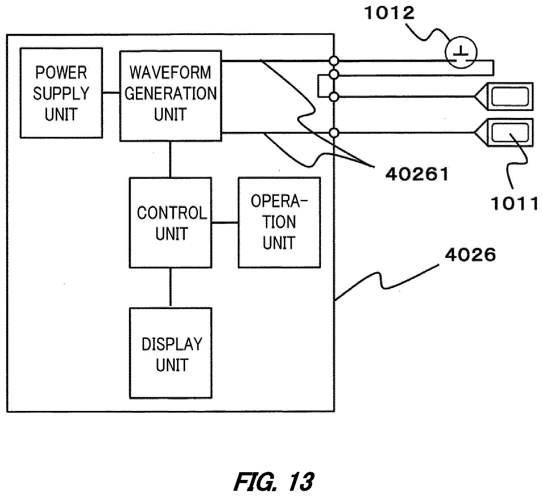

FIG. 12 is a circuit block diagram of the waterproof-type low-frequency electric stimulation device main body 402. A main body circuit unit 4026 of the device main body 402 include a power supply unit, an operation unit, a control unit, a waveform generation unit, a display unit, and the like. The waveform generation unit is mainly configured by a switching element and the like, and outputs an electric stimulation output having a unipolar or bipolar pulse waveform or the like to an electric stimulation output line 40261, in response to an instruction output from a microcomputer of the control unit. In the present example, the insulator electrode 1011 for low-frequency electric stimulation and the energization permission switch 1012 are provided, and the energization permission switch is connected in series with the electric stimulation output line 40261.

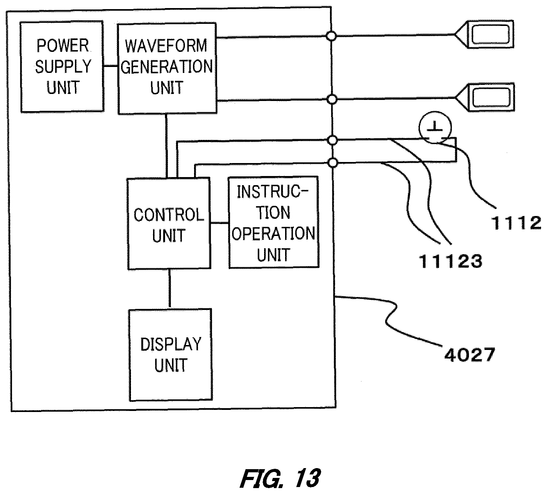

FIG. 13 is a circuit block diagram when the energization permission sensor 1112 is provided as the waterproof-type low-frequency electric stimulation device 401. The sensor core wire 11123 of the energization permission sensor 1112 is directly connected to a terminal of a microcomputer (not shown) provided in a control unit of a main body circuit unit 4027, and the microcomputer detects on/off states of the switch from a change in voltage of the terminal and controls on/off of the electric stimulation output. In this way, an output of the energization permission sensor is directly input to the control unit of the waterproof-type low-frequency electric stimulation device main body, so that it is not necessary to separately provide a switch for turning on/off the electric stimulation output. Also in a case where the energization permission sensor integrated type insulator electrode 1111 for low-frequency electric stimulation is used, a similar circuit can be used.

<Embodiment of Waterproof Bag For Push Switch-Type Low-Frequency Electric Stimulation Device>

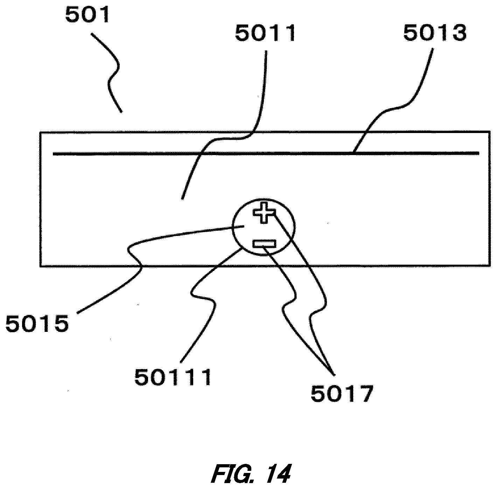

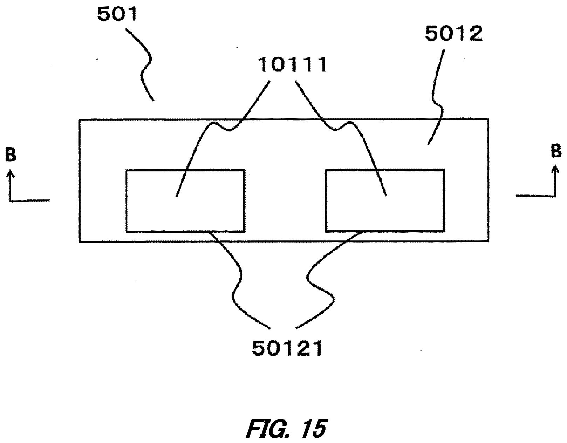

A waterproof bag for a push switch-type low-frequency electric stimulation device is a waterproof case for enabling a cordless low-frequency electric stimulation device having a push switch to be safely used in water or in a state where skin is wet by accommodating the cordless low-frequency electric stimulation device in the case. FIG. 14 is a plan view of a waterproof bag 501 for a push switch-type low-frequency electric stimulation device, as seen from an operation surface-side. In FIG. 14, it is assumed that the cordless low-frequency electric stimulation device is accommodated with an operation surface facing a front surface. FIG. 15 shows a backside of the waterproof bag 501. The waterproof bag 501 is configured by an operation surface-side sheet 5011, an electrode surface-side sheet 5012, a waterproof seal 5013, a pair of openings 50121 provided in the electrode surface-side sheet 5012, insulator electrode films 10111 bonded to shield the openings, and the like. In a case where an opaque sheet is used as the operation surface-side sheet 5011, the operation surface-side sheet 5011 is provided with an opening 50111 so as to visually recognize the operation switch and the like of the accommodated cordless low-frequency electric stimulation device from the outside, and the opening is shielded by a transparent resin sheet 5015.

When the cordless low-frequency electric stimulation device is accommodated in the waterproof bag for the push switch-type low-frequency electric stimulation device, which is then used in water, it is difficult to visually recognize the operation switch of the present device in the bag in water due to an influence of reflection resulting from an refractive index of water. Therefore, the user will find a position of the operation switch by touch of a fingertip. A protrusion 5017 is provided on a surface of the operation surface-side sheet 5011 or a surface of the transparent resin sheet 5015 at a position corresponding to the operation switch of the present device so that the position of the switch can be easily detected by the fingertip.

The waterproof bag 501 may have a structure where a third sheet (referred to as a connection sheet, not shown) for connecting the operation surface-side sheet and the electrode surface-side sheet is provided and the waterproof bag 501 bulges three-dimensionally. Alternatively, the operation surface-side sheet and/or the electrode surface-side sheet may not be a flat sheet and may be a sheet molded so as to have a three-dimensional bulge. In particular, the waterproof bag desirably has a three-dimensional shape so as to correctly fit with a three-dimensional shape of the cordless low-frequency electric stimulation device accommodated therein. Since such a three-dimensional shape eliminates unnecessary entrainment of air, when the waterproof bag is immersed in water, generation of unnecessary buoyancy can be reduced, so that it is possible to easily handle the waterproof bag in water. Also, when inserting the cordless low-frequency electric stimulation device into the waterproof bag, it is necessary to match the positions of the electrodes of the device and the positions of the insulator electrode films of the waterproof bag. However, a three-dimensional convex part of the device is fitted with a concave part of the waterproof bag, so that positional alignment is facilitated.

[Sectional Configuration]

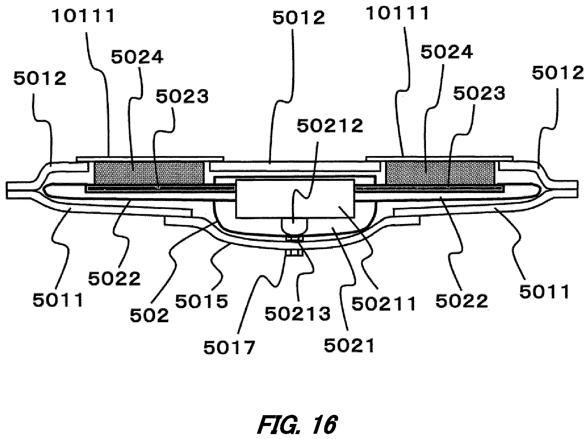

FIG. 16 is a BB sectional view in a state where the cordless low-frequency electric stimulation device 502 is accommodated in the waterproof bag 501. The cordless low-frequency electric stimulation device 502 has a main body unit 5021 and a sheet-like electrode unit 5022 extending right and left. In the main body unit 5021, a main body circuit unit 50211, an operation switch unit 50212 and the like are embedded. Electroconductive electrode sheets 5023 are exposed on the electrode unit 5022, and electroconductive adhesive gels 5024 adhere to surfaces of the electroconductive electrode sheets. The electroconductive adhesive gels adhere to the insulator electrode films 10111 through the opening portions of the electrode surface-side sheet 5012. In the present example, the operation surface-side sheet 5011 is formed with an opening, and the opening is shielded by the transparent resin sheet 5015. When the operation surface-side sheet 5011 is transparent, the opening and the transparent resin sheet 5015 are not required. In the present example, the insulator electrode film is bonded to an outer side of the electrode surface-side sheet 5012 but may be bonded to an inner side of the electrode surface-side sheet. Also, a single film of the insulator electrode film is used. However, an insulator electrode sheet (which will be described later) configured by the insulator electrode film, the electroconductive substrate and the like may also be used.

[Using Method and Fixing Method]

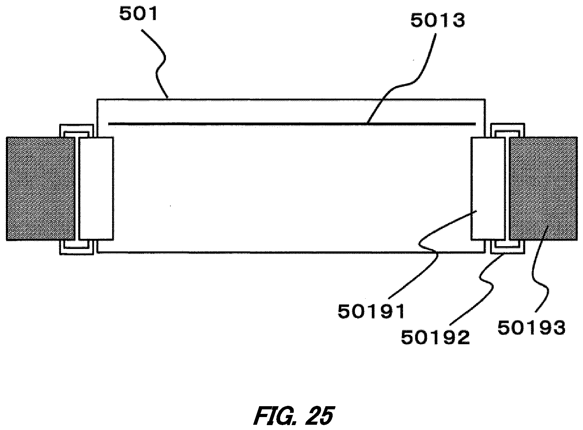

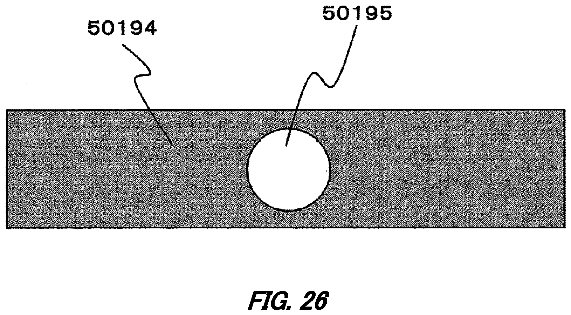

In order to use the waterproof bag 501, first, the cordless low-frequency electric stimulation device is accommodated in the waterproof bag while aligning positions so that the electroconductive adhesive gels 5024 adhering to the electrode unit are to adhere to the insulator electrode films 10111. Then, the waterproof seal 5013 is closed. The insulator electrode films 10111 are brought into contact with the skin and are then fixed with the extendible fixing belt. FIG. 25 shows an example where the fixing belt is attached to the waterproof bag 501. Thermoplastic resins 50191 are welded to both left and right end portions of the waterproof bag, thereby attaching rectangular rings 50192. An extendible fixing belt 50193 is enabled to pass through the rectangular rings, is put around the body and is then fixed by Velcro (registered trademark) or the like. Alternatively, as shown in FIG. 26, the waterproof case may be wrapped from above by using an extendible fixing belt 50194 that is a separate member and is formed with a main body unit fitting hole 50195. In the fitting hole 50195, the main body unit 5021 having a convex shape is fitted. The fixing belt may be formed of an elastic fiber or the like that is generally spread. For example, stretch fiber, stretch fabric or the like formed of polyester, polyurethane, polytrimethylene terephthalate, polybutylene terephthalate, nylon or the like may be used.

An operation switch 50213 and an operation switch unit 50212 are operated by pushing the same from above the waterproof case. Alternatively, when the cordless low-frequency electric stimulation device 502 has a communication function of using Bluetooth (registered trademark), the switches can be wirelessly operated from a smartphone and the like by using Bluetooth (registered trademark). When taking out the cordless low-frequency electric stimulation device 502 from the waterproof bag 501, the waterproof seal 5013 is opened, and the electroconductive adhesive gels 5024 are detached from the insulator electrode films 10111.

In the present example, the two insulator electrode films 10111 are separately provided. However, one insulator electrode film may cover the plurality of electroconductive electrodes. Also, the electrode surface-side sheet itself may be configured as the insulator electrode film. Also, two or more cordless low-frequency electric stimulation devices may be accommodated in one waterproof bag. Also, the number of electrodes of one cordless low-frequency electric stimulation device may be three or more. Also, in FIG. 14, the waterproof seal 5013 is provided on the operation surface-side sheet 5011 but may be provided on the electrode surface-side sheet 5012. In the present example, the cordless low-frequency electric stimulation device is accommodated in the waterproof bag. However, a low-frequency electric stimulation device having corded electrodes may also be accommodated. In this case, a cord is attached to the electrode, so that there is a degree of freedom with respect to a position at which the electrode is bonded to the insulator electrode film. The degree of freedom can be increased by increasing the number or area of the insulator electrode film. A backside of the device main body may be fixed as appropriate so that the main body of the corded low-frequency electric stimulation device does not move in the waterproof case.

[Insulator Electrode Sheet]

In a case where an electroconductive substrate is stacked on a backside of the insulator electrode film 10111 to form an insulator electrode sheet so as to reinforce mechanical strength of the insulator electrode film 10111, when the electroconductive adhesive gel 5024 of hydro polymer is used by being bonded to the electroconductive substrate, corrosion pits are formed in the metallic electroconductive substrate due to electric corrosion in a short time after energization. Such an accident does not occur if the electroconductive adhesive gel does not contain moisture. However, hydro polymer containing moisture is generally spread as the electroconductive adhesive. A three-layered insulator electrode sheet where the insulator electrode film is also formed on the backside of the electroconductive substrate is provided, so that it is possible to prevent corrosion pits while reinforcing the mechanical strength. Also, when peeling off the electroconductive adhesive gel from the insulator electrode sheet, the electroconductive adhesive gel can be easily peeled off.

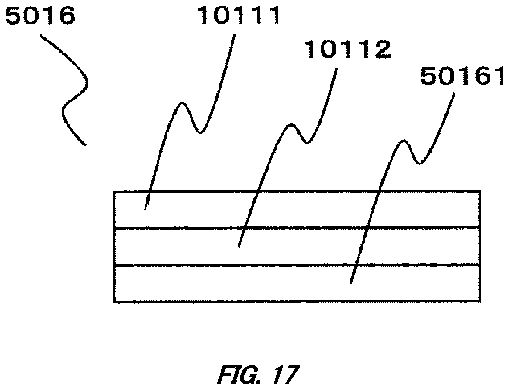

FIG. 17 is a sectional view of an insulator electrode sheet 5016. In the insulator electrode sheet, an outer surface of the electroconductive substrate 10112 is formed with the insulator electrode film 10111, and an inner surface of the electroconductive substrate is formed with an inner insulator electrode film 50161. A material of the inner insulator electrode film may be the same as the material of the insulator electrode film 10111 or a different resin material having a large relative permittivity may be used. Since it is required to implement the electrostatic capacitance shown in Table 1 over the entire insulator electrode sheet, the inner insulator electrode film 50161 needs to be thinner than the insulator electrode film 10111. Since the inner insulator electrode film is less likely to be scratched and worn in association with use, the film thickness can be reduced. When the insulator electrode sheet 5016 is used, the plurality of electroconductive electrodes cannot be covered with the one sheet, so that it is necessary to provide an independent insulator electrode sheet for each of the electroconductive electrodes.

[Material of Waterproof Bag]

For the operation surface-side sheet 5011, the electrode surface-side sheet 5012, the transparent resin sheet 5015 and the connection sheet, a general resin sheet can be used. However, a thermoplastic resin having a low water-vapor transmission ratio is particularly preferable. Specifically, polyvinyl chloride (PVC), polyethylene terephthalate (PET), ethylene-tetrafluoroethylene copolymer (ETFE), polyvinylidene chloride (PVDC), polyethylene (PE), polypropylene (PP), polyvinylidene fluoride (PVDF), tetrafluoroethylene-perfluoroalkyl vinyl ether copolymer (PFA), tetrafluoroethylene-hexafluoropropylene copolymer (FEP), polychlorotrifluoroethylene (PCTFE), polytetrafluoroethylene (PTFE), and the like may be exemplified. However, the present invention is not limited thereto.

Also, the sheet may be multi-layered. For example, a laminate sheet or the like where a PCTFE film is bonded to a soft PVC sheet to reduce the water-vapor transmission ratio may be used. Also, when a sheet where a thermoplastic resin is laminated on an aluminum foil is used, the water-vapor transmission ratio can be significantly reduced and heat transmission can also be reduced. Such sheet is generally used for a retort pouch case and the like. However, the aluminum foil laminate is opaque. Therefore, when using the aluminum foil laminate as the operation surface-side sheet, a part of the operation surface-side sheet, at which the main body unit is positioned, is formed with an opening and the opening is shielded with a transparent thermoplastic resin film, for example, PCTFE, a laminate film of PCTFE and soft PVC, or the like for a transparent window sheet so that the operation switch and the like of the main body unit can be visually recognized from an outside,. The favorable thicknesses of the operation surface-side sheet, the electrode surface-side sheet and the transparent window sheet vary depending on the materials but are desirably within a range of 30 $\mu$m to 1 mm. When the thickness is 30 $\mu$m or less, the durability is poor, and when the thickness is 1mm or greater, the flexibility is impaired.

[Waterproof Seal]

The waterproof seal preferably has a structure that can be freely openable/closable and can keep waterproofness even when immersed in water. For example, the waterproof case aLOKSAK (registered trademark) made of polyethylene available from LOKSAK Company (USA) has a seal part having a simple structure where linear convex and concave portions are fitted, but guarantees perfect waterproofness in water. There is also a fastener-type waterproof seal such as AQUA seal (registered trademark) available from YKK, Proseal (registered trademark) and the like. Such waterproof seals can be selected as appropriate.

[Bonding of Members]

The operation surface-side sheet, the electrode surface-side sheet, the connection sheet, the waterproof seal, the insulator electrode film, the transparent resin sheet and the like, which are members configuring the waterproof bag, are bonded by welding, a double-sided tape, an adhesive or the like. For example, the operation surface-side sheet and the electrode surface-side sheet are made of a thermoplastic resin, and may be bonded to configure a waterproof bag by welding. Also in a case of the aluminum foil laminate sheet, the aluminum foil laminate sheets can be welded each other by using a thermoplastic resin for the outermost layer. By using a thermoplastic resin for the insulator electrode film, the insulator electrode film can be welded with the electrode surface-side sheet. Also in a case where a composite film in which inorganic fine particles such as barium titanate are dispersed in a thermoplastic resin such as PVDF is used for the insulator electrode film, the insulator electrode film can be welded with the electrode surface-side sheet formed of the thermoplastic resin. The members to be welded are desirably made of the same thermoplastic resin. However, even when the members are made of different materials, the members can be welded by devising conditions. For example, in a case of PVDF and soft PVC, PVDF and soft PVC can be welded by sandwiching an acrylic resin having high compatibility with PVDF therebetween.

Also, the waterproof sealing can be conveniently made using a VHB (registered trademark) double-sided tape available from 3M Company. 3M VHB Y-4180 (registered trademark) can adhere to polypropylene and polyethylene that are difficult to adhere. Also in a case where a laminate film of PCTFE and soft PVC is used for the transparent window sheet, the transparent window sheet can be welded with the operation surface-side sheet. As a welding method, general methods such as pressure welding by a hot plate, ultrasonic welding, laser welding and the like can be used. In order to secure reliability of the waterproof, it is desirable to configure adhesion surfaces of the members by the thermoplastic resin and to bond the members by welding.

<Embodiment 2 of Waterproof-Type Low-Frequency Electric Stimulation Device>

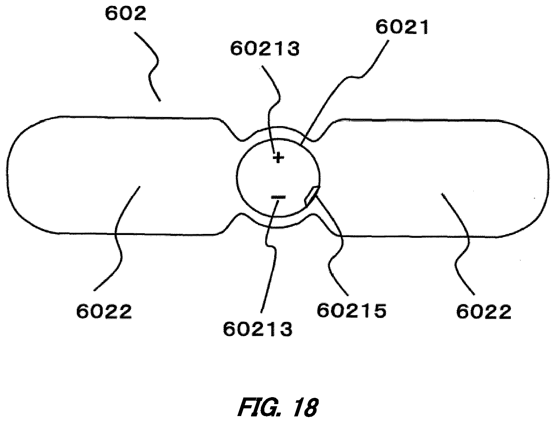

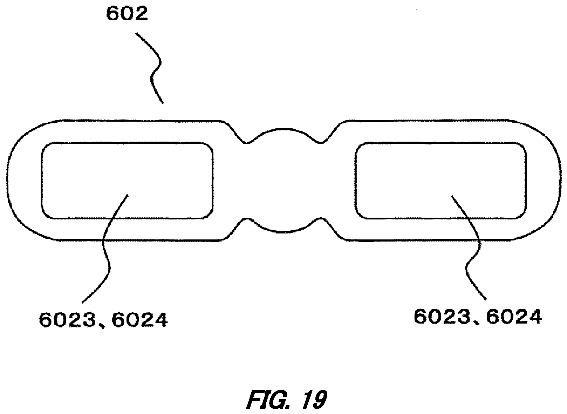

The present waterproof-type low-frequency electric stimulation device is a waterproof bag compatible type cordless low-frequency electric stimulation device having an energization permission sensor compatible function and an underwater communication function and accommodated in the above-described waterproof bag 501 for a push switch-type low-frequency electric stimulation device. FIG. 18 shows an operation surface-side of a waterproof bag compatible type cordless low-frequency electric stimulation device 602. FIG. 19 shows an electrode surface-side of the present device.

The waterproof bag compatible type cordless low-frequency electric stimulation device 602 is configured by a main body unit 6021 and a sheet-like electrode unit 6022. The main body unit is provided on its surface with an operation switch 60213 and a receptacle 60215 for charging. The user can turn on/off the operation switch 60213 by pushing the same with a finger. Also, the user may adjust an electric stimulation intensity and an electric stimulation waveform. The electrode surface-side of the sheet-like electrode unit is configured by electroconductive electrode sheets 6023 and electroconductive adhesive gels 6024 bonded to surfaces of electroconductive electrode sheets.

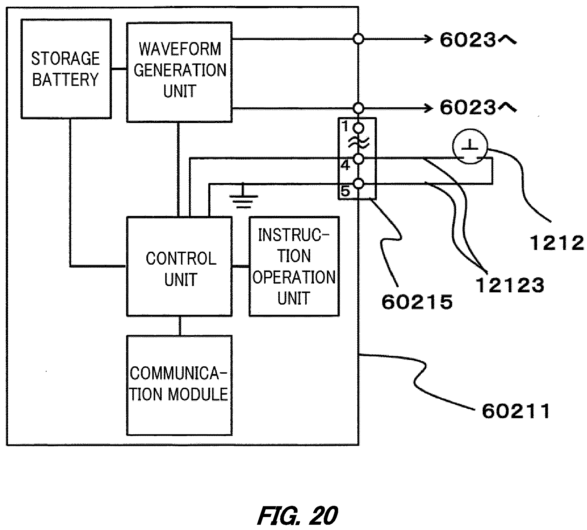

FIG. 20 is a block diagram of a main body circuit unit 60211 of the device 602. The main body circuit unit 60211 is configured by a control unit, an instruction operation unit, a communication module, a waveform generation unit, a storage battery, and the like. The instruction operation unit can be operated by the operation switch 60213. Also, an instruction operation can be performed via the communication module from an external communication terminal such as a smartphone by using a wireless communication means such as Bluetooth (registered trademark). By receiving the instruction, an instruction to generate an electric stimulation waveform is issued from the control unit to the waveform generation unit. The prepared electric stimulation waveform is output to the electroconductive electrode sheets 6023. The charging is performed by connecting a plug for charging (not shown) to the receptacle 60215 for charging. In FIG. 20, an aspect where the energization permission sensor 1212 is connected to the receptacle 60215 for charging is shown.

[Energization Permission Sensor 1212]

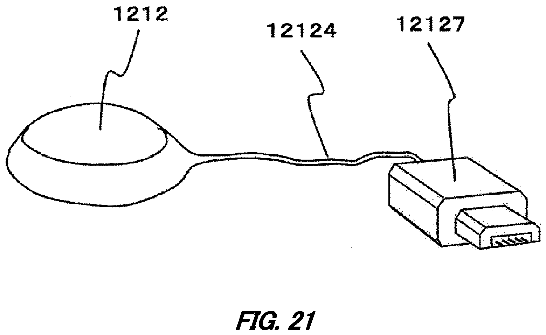

For the plug and receptacle for charging, a micro USB is generally used. The micro USB plug/receptacle has five pins. However, for a charging use, second to fourth pins are not used. Therefore, as shown in FIG. 20, sensor core wires 12123 of the energization permission sensor 1212 are connected to the fourth pin and the fifth pin which is a ground, of the micro USB. Then, the fourth pin is connected to the terminal of the microcomputer in the control unit. The microcomputer can turn on the electric stimulation output when the fourth pin is connected to the ground, and turn off the electric stimulation output when the fourth pin is open. In the present example, the fourth pin and the fifth pin are used. However, any two pins of the second to fourth pins may be used. FIG. 21 shows a configuration of the energization permission sensor 1212. An electric wire cord 12124 is attached to the energization permission sensor 1212, and the sensor core wires 12123 are inserted in the electric wire cord. A micro USB plug 12127 is connected to a tip end of the electric wire cord.

The energization permission sensor 1212 is connected to the receptacle 60215 for charging of the waterproof bag compatible type cordless low-frequency electric stimulation device 602, and is accommodated in the waterproof case 501 together with the device 602. The energization permission sensor 1212 is placed on a surface or the like of the sheet-like electrode unit 6022 of the device 602. The energization permission sensor is turned on by a pressure that is generated when the waterproof case is brought into contact with the human body and is fixed by wrapping the same around the human body with the fixing belt, and when the wrapping-fixed state by the fixing belt is released, the pressing pressure disappears and the energization permission sensor is turned off. As the internal structure of the energization permission sensor 1212, the same structure as that shown in FIG. 4 can be used. Since the energization permission sensor is accommodated in the waterproof case, the electric wire cord 12124 is not required to be a waterproof cord, and a general insulated covered electric wire can be used.

(Other Embodiments of Plug/Receptacle)

The plug that is mounted to the energization permission sensor 1212 is not necessarily limited to the micro USB. For example, a phone mini plug, a phone micro plug and the like may also be used. In this case, the main body unit 6021 of the waterproof bag compatible type cordless low-frequency electric stimulation device 602 is provided with a phone jack for a phone plug (not shown) separately from the receptacle 60215 for charging, and a wire from the phone jack is connected to the terminal of the microcomputer in the control unit. Thereby, the microcomputer can detect on/off states of the energization permission sensor.

(Other Embodiments of Energization Permission Sensor)

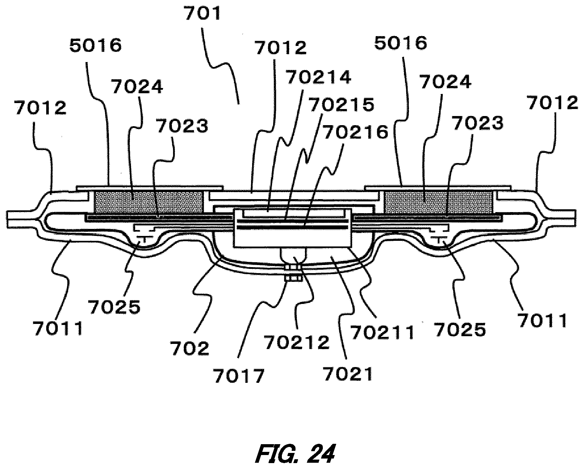

The momentary switch as the energization permission sensor may be embedded in the waterproof bag compatible type cordless low-frequency electric stimulation device 602, and may be arranged on the surface or the like of the sheet-like electrode unit 6022 so as to protrude in a convex form. A sectional structure thereof is a sectional view of another embodiment but is the same as an energization permission sensor 7025 shown in FIG. 24. By wrapping and fixing the waterproof case around the human body with the fixing belt, the switch can be turned on by a pressure received via the operation surface-side sheet of the waterproof case 501. When the fixing belt is unfastened, the pressing force disappears and the momentary switch is turned off, so that the electric stimulation output can be cut off.

[Bluetooth (registered trademark) Communication In Water]

When the cordless low-frequency electric stimulation device is attached at a position such as a back that is out of reach, it is impossible to push the operation switch arranged on the main body unit of the device. For this reason, products capable of wirelessly controlling an output by using Bluetooth (registered trademark) from an external terminal such as a smartphone is increasing. When inserting the cordless low-frequency electric stimulation device in the waterproof bag and using the same in water, there is a problem that radio waves of Bluetooth (registered trademark) are absorbed by water and the human body. In a case of radio wave intensity of Bluetooth (registered trademark) Class 2 that is generally used at present, stable transmission and reception can be performed even in water as long as a distance is about a depth of a bathtub. However, when the user holds the smartphone in user's hand and the present device is hidden in the shadow of the human body in water, the radio waves are largely absorbed inside of the human body, so that communication becomes unstable. In the meantime, in a case of Bluetooth (registered trademark) Class 1.5 (radio wave output 10 mW) or higher, stable communication can be performed even in the bathtub. Therefore, it is necessary to mount a communication module having a radio wave output of Bluetooth (registered trademark) Class 1.5 (radio wave output 10 mW) or higher in the waterproof bag compatible type cordless low-frequency electric stimulation device 602 of the present invention.

In this way, by mounting the energization permission sensor function and the communication function that can be used in water onto existing cordless low-frequency electric stimulation devices, the waterproof bag compatible type cordless low-frequency electric stimulation device 602 is completed, and by accommodating the present device 602 in the push switch-type waterproof case 501 for a low-frequency electric stimulation device, a waterproof-type low-frequency electric stimulation device 601 (not shown) of the present embodiment 2 is completed.

<Embodiment 3 of Waterproof-Type Low-Frequency Electric Stimulation Device>

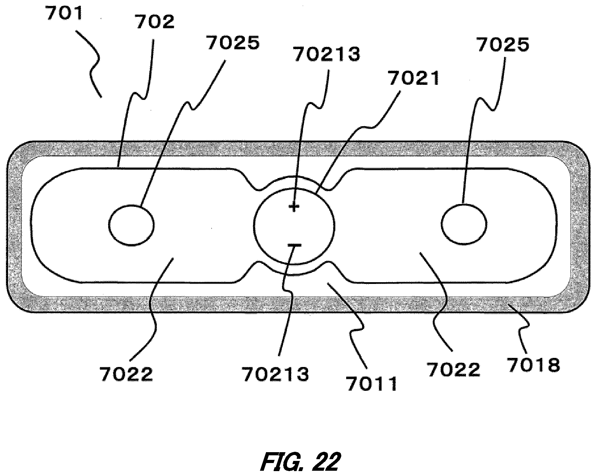

A waterproof-type low-frequency electric stimulation device 701 of the present embodiment has such a structure that a waterproof seal compatible type cordless low-frequency electric stimulation device 702, in which the wireless charging function, the energization permission sensor and the underwater communication function are embedded in a usual cordless low-frequency electric stimulation device, is hermetically sealed by a resin film. FIG. 22 is a plan view of an operation surface-side. The waterproof seal compatible type cordless low-frequency electric stimulation device 702 is configured by a main body unit 7021 and a sheet-like electrode unit 7022, and the main body unit 7021 is provided with an operation switch 70213. Further, an energization permission sensor 7025 is embedded so as to protrude from a surface of the electrode unit.

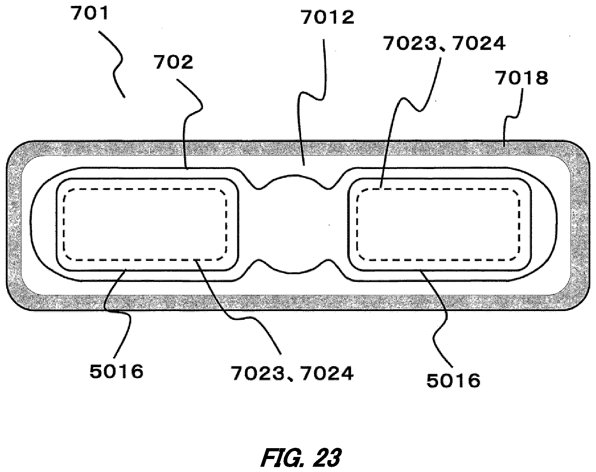

FIG. 23 shows an electrode surface-side of the device 701. A reference sign 7023 indicates an electroconductive electrode sheet, and a reference sign 7024 indicates an electroconductive adhesive gel that adheres onto the electroconductive electrode sheet. The insulator electrode sheet 5016 is bonded so as to shield an opening of the electrode surface-side sheet 7012. The waterproof seal compatible type cordless low-frequency electric stimulation device 702 are covered by an operation surface-side sheet 7011 and an electrode surface-side sheet 7012, and peripheral edge parts 7018 of both the sheets are hermetically sealed by welding, a double-sided tape or the like.

FIG. 24 is a sectional view of the waterproof-type low-frequency electric stimulation device 701. The electrode surface-side sheet 7012 is formed with an opening portion, and the opening portion is shielded by the insulator electrode sheet 5016. The electroconductive adhesive gel 7024 adheres onto the electroconductive electrode sheet 7023 of the waterproof seal compatible type cordless low-frequency electric stimulation device 702, and an outer surface of the adhesive gel adheres to the insulator electrode sheet 5016. Note that, the insulator electrode film 10111 may also be used, instead of the insulator electrode sheet 5016. Also, in a case where an electroconductive adhesive gel for which hydro polymer is not used is used for the electroconductive adhesive gel, a two-layered insulator electrode sheet configured by the insulator electrode film 10111 and the metallic electroconductive substrate 10112 may be used. Also in a case where an electroconductive adhesive is used instead of the electroconductive adhesive gel 7024, the two-layered insulator electrode sheet can be used.

[Wireless Charging Function]

A bottom surface of a main body circuit unit 70211 of the waterproof seal compatible type cordless low-frequency electric stimulation device 702 is provided with a power receiving coil 70214 for wirelessly receiving charging energy resulting from electromagnetic induction or magnetic field resonance from an outside, a magnetic sheet 70215 for preventing leakage of a magnetic flux, and a power receiving circuit substrate 70216, so that a storage battery (not shown) in the main body circuit unit can be wirelessly charged from the outside. Products that can be generally available can be used for components and units relating to wireless power supplying. For example, a 2-watt power receiving coil made by TDK has a diameter of 2 cm, and a thickness integrated with the magnetic sheet is 1 mm or smaller. Also, the power receiving circuit substrate 70216 may be fitted to have substantially the same substrate size. Charging can be performed wirelessly by bringing the power receiving coil 70214 close to a charger (not shown) having a power transmission coil provided therein and magnetically coupling the power receiving coil and the charger. When an aluminum foil laminate film is used for the electrode surface-side sheet 7012, the aluminum foil is interposed between the power receiving coil 70214 and the power transmission coil (not shown). Since power supplying is performed by high-frequency alternating current, an eddy current is generated in the aluminum foil, so that heat is generated and power supplying energy is lost. Therefore, in this case, a part of the electrode surface-side sheet facing the power receiving coil 70214 is provided with an opening (not shown), and the opening is shielded by a resin sheet (not shown).

[Energization Permission Sensor]

In FIG. 24, the energization permission sensor 7025 is embedded in the surface of the sheet-like electrode unit of the waterproof seal compatible type cordless low-frequency electric stimulation device 702. Since the sheet-like electrode unit 7022 is covered with a sheet-like elastomer or the like, the pressing resulting from the wrapping around the human body can push the momentary switch of the energization permission sensor 7025. As the structure of the energization permission sensor, the same structure as that shown in FIG. 4 can be used. The sensor core wire of the energization permission sensor is connected to the terminal of the microcomputer in the main body circuit unit 70211, and when the sensor switch is released from a pressed state, an unused state is determined and the electric stimulation output is cut off.

[Bluetooth (registered trademark) Communication In Water]

Since the main body circuit unit 70211 is mounted with the communication module (not shown) and has the radio wave output of Bluetooth (registered trademark) Class 1.5 (10 mW) or higher, stable communication can be performed with the smartphone or the like even in the bathtub.

With the above-described configuration, it is possible to implement the waterproof-type low-frequency electric stimulation device 701 capable of being charged and having excellent waterproofness. In the above descriptions, the number of the electrodes of the waterproof seal compatible type cordless low-frequency electric stimulation device 702 is two. However, the number of the electrodes may also be three or more. By increasing the number of the electrodes, it is possible to apply electric stimulation over a wider range. Also, the waterproof seal compatible type cordless low-frequency electric stimulation device that is hermetically sealed may be two or more, not one. In the above descriptions, each of the insulator electrode films 10111 is independently arranged according to the position of the electroconductive adhesive gel. However, one insulator electrode film may also cover a plurality of electroconductive electrodes. Also, the electrode surface-side sheet itself may be the insulator electrode film.

[Other Embodiments of Waterproof Seal Compatible Type Cordless Low-Frequency Electric Stimulation Device 702]

The operation surface-side sheet 7011 and the electrode surface-side sheet 7012 may be excluded by configuring the waterproof seal compatible type cordless low-frequency electric stimulation device 702 itself as a waterproof structure and by using the insulator electrode film or the insulator electrode sheet as the electrode part. Since the energization permission sensor and the wireless charging function are embedded, there is no receptacle, an opening/closing mechanism and the like, and consequently, it is easy to configure the waterproof structure. As an example of the waterproof structure, the waterproof seal compatible type cordless low-frequency electric stimulation device 702 is configured to have a structure covered with the thermoplastic resin or thermoplastic elastomer, so that the device can be welded with the insulator electrode film or the insulator electrode sheet.

<Embodiment 4 of Waterproof-Type Low-Frequency Electric Stimulation Device>

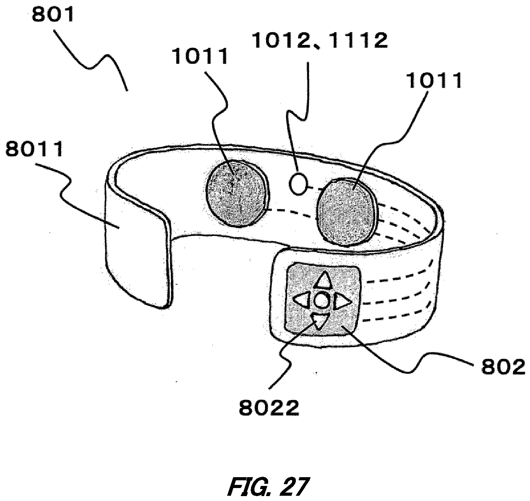

FIG. 27 shows a waterproof-type low-frequency electric stimulation device 801 integrated with a support body. In the present device, a waterproof-type low-frequency electric stimulation device main body 802, the insulator electrodes 1011 for low-frequency electric stimulation, and the energization permission switch 1012 or the energization permission sensor 1112 are integrally attached to a fixing belt support body 8011. A reference sign 8022 indicates an operation switch. The broken line in FIG. 27 indicates waterproof cords inserted in the fixing belt support body. The waterproof-type low-frequency electric stimulation device main body 802 has the same function as the waterproof-type low-frequency electric stimulation device main body 402 but has waterproof performance that allows it to be immersed in water. Also, the main body may have a communication function having a radio wave output of Bluetooth (registered trademark) Class 1.5 or higher so that it can be operated from an external terminal such as a smartphone. The insulator electrode 1011 and the energization permission switch 1012 or the energization permission sensor 1112 may be fixed by Velcro (registered trademark) or the like with a degree of freedom so that the user can freely adjust the adhesion positions. Instead of the insulator electrode for low-frequency electric stimulation and the energization permission sensor, the energization permission sensor integrated type insulator electrode for low-frequency electric stimulation may also be used.

<Embodiment 5 of Waterproof-Type Low-Frequency Electric Stimulation Device>

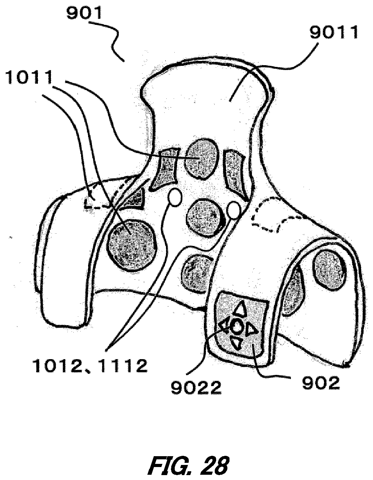

FIG. 28 shows a waterproof-type low-frequency electric stimulation device 901 having a shoulder-mounted support body 9011 that can be used for low-frequency electric stimulation to shoulders, a neck and a back. A plurality of the insulator electrodes 1011 is provided, and two energization permission sensors 1012 or two energization permission sensors 1112 are provided. A reference sign 902 indicates a waterproof-type low-frequency electric stimulation device main body, and a reference sign 9022 indicates an operation switch of the device main body. The two switches or sensors are electrically connected in parallel, and when any one is turned on, the electric stimulation output is turned on, and when both the energization permission sensors become off at the same time, the electric stimulation output is turned off. The shape of the support body is not limited thereto, and the support body can freely adopt a shape in conformity to the shape of the human body, according to the contact part with the human body. Instead of the insulator electrode for low-frequency electric stimulation and the energization permission sensor, the energization permission sensor integrated type insulator electrode for low-frequency electric stimulation may also be used.

As the fixing belt support body 8011, an elastic belt or the like configured using stretch fiber or stretch fabric made of polyester, polyurethane, polytrimethylene terephthalate, polybutylene terephthalate, nylon or the like can be used. In the case of the shoulder-mounted support body 9011, in addition to being composed of the extendible elastic fiber, a shell may be composed of a material having elasticity and strength and a soft material that can conform to the three-dimensional shape of the human body may be lined on an inner side of the shell. In order to configure a skeleton and a shape, a fiber-reinforced plastic, a resin such as soft vinyl chloride, elastomers and the like can be used. In order to configure the lining, a three-dimensional structure of a random loop of continuous filaments made of a resin-based elastomer, a resin foaming body, a net-like elastic body in which fibers are knitted three-dimensionally or a thermoplastic elastomer having rubber elasticity can be used.

INDUSTRIAL APPLICABILITY

According to the present invention, it is possible to safely use the low-frequency electric stimulation device such as a low-frequency treatment device, an EMS device and the like even when bathing or in water. When bathing, it is possible to improve the low-frequency treatment effects by a synergetic effect of the low-frequency electric stimulation effect and the hyperthermic effect. Also, it is possible to effectively utilize the bathing time. Also, since the low-frequency electric stimulation device can be safely used even in a state where skin is wet, new usage such as performing EMS while playing sports involving sweating is possible. The low-frequency electric stimulation device can be used not only at home but also in hot springs, spas, gyms, nursing care facilities, treatment centers and the like.

EXPLANATION OF REFERENCES

101: waterproof case for non-waterproof-type low-frequency electric stimulation device

1011: insulator electrode for low-frequency electric stimulation

10111: insulator electrode film

10112: electroconductive substrate

10113: electric stimulation output core wire

10114: waterproof cord

10115: insulating covering

1012: energization permission switch

10121: lower electrode

10122: inversion spring upper electrode

10123: switch core wire

10124: waterproof cord

10125: flexible waterproof covering

10126: plunger

1013: terminal connection part

10131: connection plug

10132: electric stimulation output line

1014: lower case

1015: upper lid

10151: waterproof switch

1017: buckle

1018: O-ring

1019: hinge

102: non-waterproof-type low-frequency electric stimulation device main body

1021: output jack part

1022: operation switch

1111: energization permission sensor integrated type insulator electrode for low-frequency electric stimulation

1112: energization permission sensor

11123: sensor core wire

1113: sensor circuit

11131: power supply switch

11132: battery

11134: photo-coupler

11135: N-channel MOSFET

11136: LED lamp

1212: energization permission sensor (plug is attached)

12123: sensor core wire

12124: electric wire cord

12127: micro USB plug

201: waterproof case (film window) for non-waterproof-type low-frequency electric stimulation device

20151, 20155: transparent resin film

20152, 20153, 20154: opening

202: non-waterproof-type low-frequency electric stimulation device main body

2022: operation switch

301: waterproof case (resin sheet case) for non-waterproof-type low-frequency electric stimulation device

3011: bag made of resin

3012: waterproof seal

302: non-waterproof-type low-frequency electric stimulation device main body

3022: operation switch

401: waterproof-type low-frequency electric stimulation device (embodiment 1)

402: waterproof-type low-frequency electric stimulation device main body

4022: operation switch

4023: power supply switch

4024: display unit

4025: waterproof cord collection part

4026, 4027: main body circuit unit

40261: electric stimulation output line

501: waterproof bag for push switch-type low-frequency electric stimulation device

5011: operation surface-side sheet

50111, 50121: opening

5012: electrode surface-side sheet

5013: waterproof seal

5015: transparent resin sheet

5016: insulator electrode sheet

50161: inner insulator electrode film

5017: protrusion

50191: thermoplastic resin

50192: rectangular ring

50193: extendible fixing belt

50194: extendible fixing belt of separate body

50195: main body unit fitting hole

502: cordless low-frequency electric stimulation device

5021: main body unit

50211: main body circuit unit

50212: operation switch unit

50213: operation switch

5022: sheet-like electrode unit

5023: electroconductive electrode sheet

5024: electroconductive adhesive gel

601: waterproof-type low-frequency electric stimulation device (embodiment 2) (not shown)

602: waterproof bag compatible type cordless low-frequency electric stimulation device

6021: main body unit

60211: main body circuit unit

60213: operation switch

60215: receptacle for charging

6022: sheet-like electrode unit

6023: electroconductive electrode sheet

6024: electroconductive adhesive gel

701: waterproof-type low-frequency electric stimulation device (embodiment 3)

7011: operation surface-side sheet

7012: electrode surface-side sheet

7017: protrusion

7018: peripheral edge part

702: waterproof seal compatible type cordless low-frequency electric stimulation device

7021: main body unit

70211: main body circuit unit

70212: operation switch unit

70213: operation switch

70214: power receiving coil

70215: magnetic sheet

70216: power receiving circuit substrate

7022: sheet-like electrode unit

7023: electroconductive electrode sheet

7024: electroconductive adhesive gel

7025: energization permission sensor

801: waterproof-type low-frequency electric stimulation device (embodiment 4) (fixing belt integrated type)

8011: fixing belt support body

802: waterproof-type low-frequency electric stimulation device main body

8022: operation switch

901: waterproof-type low-frequency electric stimulation device (embodiment 5) (shoulder-mounted integrated type)

9011: shoulder-mounted support body

902: waterproof-type low-frequency electric stimulation device main body

9022: operation switch

**Claims**

1.  A waterproof case for a non-waterproof-type low-frequency electric stimulation device comprising:

    at least a pair of insulator electrodes for low-frequency electric stimulation; and
    a terminal connection part for electrically connecting the electrodes and electric stimulation output lines of a non-waterproof-type low-frequency electric stimulation device accommodated in the waterproof case.

2.  A waterproof bag for a push switch-type low-frequency electric stimulation device, wherein

    the waterproof bag has a freely openable/closable waterproof seal part,
    at least one opening is formed on a surface of the bag, and
    the opening is shielded with an insulator electrode film or an insulator electrode sheet having an electrostatic capacitance of $0.36 \text{ nF/cm}^2$ or greater.

3.  A waterproof-type low-frequency electric stimulation device comprising:

    at least a pair of insulator electrodes for low-frequency electric stimulation; and
    at least one energization permission switch or energization permission sensor.

4.  An insulator electrode film for low-frequency electric stimulation having an electrostatic capacitance of $0.36 \text{ nF/cm}^2$ or greater, wherein
    the insulator electrode film has fine particles of an inorganic oxide having a large relative permittivity and dispersed by 10 to 70 vol% in a resin.

5.  The insulator electrode film for low-frequency electric stimulation according to Claim 4, wherein the resin is a resin of one kind selected from polyvinylidene fluoride (PVDF) monopolymer, polyvinylidene fluoride (PVDF)-based copolymer and polyvinylidene fluoride (PVDF)-based terpolymer or a resin consisting of a mixture of two or more kinds selected from the monopolymer, the copolymer and the terpolymer.

6.  An insulator electrode for low-frequency electric stimulation comprising:

    the insulator electrode film for low-frequency electric stimulation according to Claim 4 or 5;
    an electroconductive substrate formed on a backside of the insulator electrode film;
    an electric stimulation output core wire electrically connected to the electroconductive substrate;
    a waterproof cord for covering the electric stimulation output core wire; and
    an insulating covering for covering exposed live parts of the electroconductive substrate and the electric stimulation output core wire and portions of the insulator electrode film and the waterproof cord.

**FIG. 1**

**FIG. 2**

1011  10111          10113        10114

10112    10115

*FIG. 3*

**1012、1112**

**10125** **10126**

**10121** **10122** **10123** **10124**

*FIG. 4*

FIG. 5

**FIG. 6**

1111

1112

10114

10113 11123

*FIG. 7*

*FIG. 8*

**FIG. 9**

*FIG. 10*

EP 3 815 739 A1

**FIG. 11**

FIG. 13

FIG. 13

501

5011

5013

5015

50111

5017

FIG. 14

*FIG. 15*

FIG. 16

5016    10111
                10112
                        50161

**FIG. 17**

FIG. 18

602

6023、6024                    6023、6024

*FIG. 19*

FIG. 20

1212     12124     12127

**FIG. 21**

*FIG. 22*

*FIG. 23*

**FIG. 24**

501                 5013

50191        50192   50193

*FIG. 25*

**FIG. 26**

FIG. 27

**FIG. 28**

FIG. 29

*FIG. 30*

*FIG. 31*

<div style="text-align:center">**INTERNATIONAL SEARCH REPORT**</div>

| International application No. |
|---|
| PCT/JP2019/019877 |

| A. CLASSIFICATION OF SUBJECT MATTER |
|---|
| Int.Cl. A61N1/36(2006.01)i, A61N1/04(2006.01)i |

According to International Patent Classification (IPC) or to both national classification and IPC

| B. FIELDS SEARCHED |
|---|
| Minimum documentation searched (classification system followed by classification symbols)<br>Int.Cl. A61N1/00-1/44 |

Documentation searched other than minimum documentation to the extent that such documents are included in the fields searched
```
     Published examined utility model applications of Japan       1922-1996
     Published unexamined utility model applications of Japan     1971-2019
     Registered utility model specifications of Japan             1996-2019
     Published registered utility model applications of Japan     1994-2019
```

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| Y<br>A | JP 2014-501134 A (ZOLL MEDICAL CORPORATION) 20 January 2014, paragraphs [0035]-[0037], fig. 2A & WO 2012/082547 A1, specification, page 13, line 22 to page 15, line 23, fig. 2A | 1, 3<br>2, 4-6 |
| Y<br>A | US 5169380 A (BRENNAN, M. J. W.) 08 December 1992, column 6, line 55 to column 7, line 17, column 7, line 44 to column 8, line 37, fig. 1-3, 5, 6 & EP 0344920 A1 | 1, 3<br>2, 4-6 |

☒ Further documents are listed in the continuation of Box C.　　　☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 04.07.2019 | 16.07.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

<div style="text-align:center">56</div>

**INTERNATIONAL SEARCH REPORT**

| International application No. |
|---|
| PCT/JP2019/019877 |

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 3044891 U (HOMER ION LAB CO., LTD.) 16 January 1998, paragraphs [0001], [0005], [0012], fig. 1 (Family: none) | 3<br>1-2, 4-6 |
| A | JP 60-220075 A (NIHON RIKO MEDICAL CORPORATION) 02 November 1985, page 2, upper right column, line 1 to page 4, upper left column, line 8, fig. 1-4 & JP 3-1029 B2 | 1-6 |
| A | JP 2006-513739 A (STANDEN LTD.) 27 April 2006, paragraphs [0050]-[0058], fig. 5-7 & WO 2004/030760 A2, specification, page 37, line 1 to page 42, line 17, fig. 5-7 | 1-6 |

Form PCT/ISA/210 (continuation of second sheet) (January 2015)

## INTERNATIONAL SEARCH REPORT

| International application No. |
|---|
| PCT/JP2019/019877 |

**Box No. II    Observations where certain claims were found unsearchable (Continuation of item 2 of first sheet)**

This international search report has not been established in respect of certain claims under Article 17 (2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
[see extra sheet]

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.

PCT/JP2019/019877

(Continuation of Box No. III)

Document 2: US 5169380 A (BRENNAN, M. J. W.) 08 December 1992, column 6, line 55 to column 7, line 17, column 7, line 44 to column 8, line 37, fig. 1-3, 5, 6
& EP 0344920 A1

The claims are classified into the two inventions below.

(Invention 1) Claims 1-3
Claims 1-3 have the special technical feature of "waterproofing" of "a low frequency electrical stimulus device" provided with "an insulator electrode", and thus are classified as invention 1.

(Invention 2) Claims 4-6
Claims 4-6 have the common technical feature between these claims and claim 1 classified as invention 1 of "an insulator electrode for a low frequency electrical stimulus".
However, this technical feature, which does not make a contribution over the prior art in light of the disclosure of document 2 (in particular, refer to column 6, line 55 to column 7, line 17, and column 7, line 44 to column 8, line 37, and fig. 1-3, 5, and 6), cannot be considered a special technical feature.
In view of this, there are not the same or corresponding special technical features between claims 4-6 and claim 1.
Furthermore, claims 4-6 do not depend from claim 1.
In addition, claims 4-6 are not substantially identical to or similarly closely related to any of the claims classified as invention 1.
Accordingly claims 4-6 cannot be identified as invention 1.
Meanwhile, claims 4-6 have the special technical feature of "an insulator electrode film having a capacitance of 0.36 $nF/cm^2$ or more, wherein, in the insulator electrode film, fine particles of an inorganic oxide having a high relative permittivity are, in a dispersed manner, contained 10-70% by volume in a resin"; thus these claims are classified as invention 2.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009136472 A
- JP H07136286 B
- JP 4057409 B

- JP 12367 A
- US 8170684 B

**Non-patent literature cited in the description**

- *Medical Electronics and Biological Engineering,* June 1973, vol. 11 (3), 161